# EUROPEAN PATENT SPECIFICATION

(11) **EP 2 403 434 B1**
(45) Date of publication and mention of the grant of the patent: **26.04.2017**
(21) Application number: 10746859.7
(22) Date of filing: 25.02.2010
(51) Int. Cl.: A61F 2/30

(54) **PATIENT-ADAPTED AND IMPROVED ORTHOPEDIC IMPLANTS**
PATIENTENADAPTIERTE UND VERBESSERTE ORTHOPÄDISCHE IMPLANTATE
IMPLANTS ORTHOPÉDIQUES AMÉLIORÉS ET ADAPTÉS AU PATIENT

(30) Priority: 24.06.2009 US 269405 P; 04.11.2009 US 280493 P; 31.07.2009 US 273216 P; 25.02.2009 US 155362 P; 18.12.2009 US 284458 P; 26.08.2009 US 275174 P
(43) Date of publication of application: 11.01.2012
(73) Proprietor: ConforMIS, Inc., Bedford, MA 01730 (US)
(72) Inventor: BOJARSKI, Raymond, Attleboro MA 02703 (US); LANG, Philipp, Lexington MA 02421 (US); CHAO, Nam, Marlborough MA 01752 (US); FITZ, Wolfgang, Sherborn MA 01770 (US); SLAMIN, John, Wretham MA 02093 (US); STEINES, Daniel, Lexington MA 02421 (US)
(74) Representative: Grund, Martin
(86) International application number: PCT/US2010/025459
(87) International publication number: WO 2010/099353

(56) References cited:
- EP-A1- 1 886 640
- EP-A2- 1 074 229
- WO-A2-03/051210
- WO-A2-2006/058057
- US-A1- 2007 118 055
- US-A1- 2007 255 288
- US-A1- 2008 009 950

## Description

### TECHNICAL FIELD

The invention relates to improved and/or patient-adapted (e.g., patient-specific and/or patient-engineered) orthopedic implants and guide tools, as well as related methods, designs and models.

### BACKGROUND

Generally, a diseased, injured or defective joint, such as, for example, a joint exhibiting osteoarthritis, has been repaired using standard off-the-shelf implants and other surgical devices. Specific off-the-shelf implant designs have been altered over the years to address particular issues. However, in altering a design to address a particular issue, historical design changes frequently have created one or more additional issues for future designs to address. Collectively, many of these issues have arisen from one or more differences between a patient's existing or healthy joint anatomy and the corresponding features of an implant component.
EP 1886640 A1 describes a method for adapting an implant to the shape of a bone as determined by an imaging method. US 2007/255288 A1 describes methods for determining the contour of a resected bone surface and assessing a fit of a prosthesis thereon and for designing customized prostheses. US 2007/118055 A1 describes the selection, design and creation of customized medical implants comprising a custom attribute, which can attach to an anatomic structure attribute of a patient. EP 1074229 A2 describes a method for planning implantation surgery based on tomographic images, where a knee joint is corrected virtually and corresponding cuts to the bone and to the implant are designed based on the virtual image.

### SUMMARY

The invention is defined by the appended claims.

The patient-adapted (e.g., patient-specific and/or patient-engineered) implant components described herein can be selected (e.g., from a library), designed (e.g., preoperatively designed including, optionally, manufacturing the components or tools), and/or selected and designed (e.g., by selecting a blank component or tool having certain blank features and then altering the blank features to be patient-adapted). Moreover, related methods, such as designs and strategies for resectioning a patient's biological structure also can be selected and/or designed. For example, an implant component bone-facing surface and a resectioning strategy for the corresponding bone-facing surface can be selected and/or designed together so that an implant component's bone-facing surface matches the resected surface.

Patient-adapted features of an implant component, guide tool or related method can be achieved by analyzing imaging test data and selecting and/or designing (e.g., preoperatively selecting from a library and/or designing) an implant component, a guide tool, and/or a procedure having a feature that is matched and/or optimized for the particular patient's biology. The imaging test data can include data from the patient's joint, for example, data generated from an image of the joint such as x-ray imaging, cone beam CT, digital tomosynthesis, and ultrasound, a MRI or CT scan or a PET or SPECT scan, is processed to generate a varied or corrected version of the joint or of portions of the joint or of surfaces within the joint. Disclosed are method and devices to create a desired model of a joint or of portions or surfaces of a joint based on data derived from the existing joint. For example, the data can also be used to create a model that can be used to analyze the patient's joint and to devise and evaluate a course of corrective action. The data and/or model also can be used to design an implant component having one or more patient- specific features, such as a surface or curvature.

Disclosed is a pre-primary articular implant component that includes (a) an outer, joint-facing surface and an inner, bone-facing surface. The outer, joint-facing surface can include a bearing surface. The inner joint facing surface can include one or more patient-engineered bone cuts selected and/or designed from patient-specific data. The patient-engineered bone cuts can be selected and/or designed from patient-specific data to minimize the amount of bone resected in one or more corresponding predetermined resection cuts. The patient-engineered bone cuts can substantially negatively-match one or more predetermined resection cuts. The predetermined resection cuts can be made at a first depth that allows, in a subsequent procedure, removal of additional bone to a second depth required for a traditional primary implant component. In addition, the pre-primary articular implant component can be a knee joint implant component, a hip joint implant component, a shoulder joint implant component, or a spinal implant component. For example, the pre-primary articular implant component can be a knee joint implant component, such as a femoral implant component. Moreover, the pre-primary articular implant component can include six or more (e.g., six, seven, eight, nine, ten, eleven, twelve, thirteen, or more) patient-engineered bone cuts on its bone facing surface.

The pre-primary articular implant component can include an implant component thickness in one or more regions that is selected and/or designed from patient-specific data to minimize the amount of bone resected. The one or more regions comprises the implant component thickness perpendicular to a planar bone cut and between the planar bone cut and the joint-surface of the implant component.

Disclosed are methods for minimizing resected bone from, and/or methods for making an articular implant for, a single patient in need of an articular implant replacement procedure. These methods can include (a) identifying unwanted tissue from one or more images of the patient's joint; (b) identifying a combination of resection cuts and implant component features that remove the unwanted tissue and also provide maximum bone preservation; and (c) selecting and/or designing for the patient a combination of resection cuts and implant component features that provide removal of the unwanted tissue and maximum bone preservation. In certain embodiments, the unwanted tissue is diseased tissue or deformed tissue.

Step (c) can include designing for the single patient a combination of resection cuts and implant component features that provide removal of the unwanted tissue and maximum bone preservation. Designing can include manufacturing. Moreover, the implant component features in step (c) can include one or more of the features selected from the group consisting of implant thickness, bone cut number, bone cut angles, and/or bone cut orientations.

A measure of bone preservation can include total volume of bone resected, volume of bone resected from one or more resection cuts, volume of bone resected to fit one or more implant component bone cuts, average thickness of bone resected, average thickness of bone resected from one or more resection cuts, average thickness of bone resected to fit one or more implant component bone cuts, maximum thickness of bone resected, maximum thickness of bone resected from one or more resection cuts, maximum thickness of bone resected to fit one or more implant component bone cuts.

A minimum implant component also can be established. For example, step (a) also can include identifying a minimum implant component thickness for the single patient. Step (b) also can include identifying a combination of resection cuts and/or implant component features that provide a minimum implant thickness determined for the single patient. Step (c) also can include selecting and/or designing the combination of resection cuts and/or implant component features that provides at least a minimum implant thickness for the single patient. The minimum implant component thickness is based on one or more of femur and/or condyle size or patient weight.

The articular implant component can be a knee joint implant component, a hip joint implant component, a shoulder joint implant component, or a spinal implant component. For example, the pre-primary articular implant component can be a knee joint implant component, such as a femoral implant component.

Disclosed is a femoral implant component that includes (a) a joint-facing surface comprising a bearing surface portion, and (b) a bone-facing surface comprising six or more bone cuts.

Disclosed is a femoral implant component that includes (a) a joint-facing surface comprising a bearing surface portion, and (b) a bone-facing surface comprising a distal bone cut having two or more planar facets or portions that are non-coplanar with each other. The two or more facets or portions can be non-parallel with each other. The first of the two or more facets or portions can be on a lateral condyle bone- facing surface and the second can be on a medial condyle bone- facing surface.

Disclosed is a femoral implant component that includes (a) a joint-facing surface comprising a bearing surface portion, and (b) a bone-facing surface comprising an anterior bone cut having two or more planar facets or portions that are non-coplanar with each other. The two or more planar facets or portions can be non-parallel with each other.

Disclosed is a femoral implant component that includes (a) a joint-facing surface comprising a bearing surface portion, and (b) a bone-facing surface comprising a posterior bone cut having two or more facets or portions that are non-parallel with each other. The first of the two or more facets or portions can be on a lateral condyle bone-facing surface and the second can be on a medial condyle bone- facing surface.

Disclosed is a femoral implant component that includes (a) a joint-facing surface comprising a bearing surface portion, and (b) a bone-facing surface comprising an anterior chamfer bone cut having two or more planar facets or portions that are non-coplanar with each other. The two or more planar facets or portions can be non-parallel with each other. The first of the two or more facets or portions can be on a lateral condyle bone-facing surface and the second can be on a medial condyle bone- facing surface.

Disclosed is a femoral implant component that includes (a) a joint-facing surface comprising a bearing surface portion, and (b) a bone-facing surface comprising a posterior chamfer bone cut having two or more facets or portions that are non-parallel with each other. The first of the two or more facets or portions can be on a lateral condyle bone-facing surface and the second can be on a medial condyle bone- facing surface.

Disclosed is a femoral implant component that includes (a) a joint-facing surface comprising lateral and medial condylar surface portions, and (b) a bone-facing surface comprising an anterior bone cut, wherein the distance between the anterior bone cut and the lateral condylar surface portion is different from the distance between the anterior bone cut and the medial condylar surface portion. The two or more facets or portions can be substantially non-parallel.

Disclosed is a femoral implant component that includes (a) a lateral and medial condylar portions, and (b) a bone-facing surface comprising a distal bone cut facet that is asymmetric about its center line in a sagittal plane. The asymmetric distal bone cut facet can lie on the bone-facing surface of the lateral condyle. The femoral implant component of claim 38, wherein the asymmetric distal bone cut facet lies on the bone-facing surface of the medial condyle.

Disclosed is a femoral implant component that includes a femoral implant component having a bone-facing surface comprising one or more bone cuts, wherein at least one of the one or more bone cuts comprises two planar bone cut facets separated by at least one step cut. The step cut can be substantially perpendicular to at least one of the bone cut facets. In certain embodiments, the step cut can rise or fall at about 30 degrees or more from at least one of the bone cut facet planes.

It is to be understood that the features of the various embodiments described herein are not mutually exclusive and may exist in various combinations and permutations.

### BRIEF DESCRIPTION OF THE DRAWINGS

The foregoing and other objects, aspects, features, and advantages of embodiments will become more apparent and may be better understood by referring to the following description, taken in conjunction with the accompanying drawings, in which:
**FIG. 1** is a flow chart illustrating a process that includes selecting and/or designing an initial patient-adapted implant, in accordance with an embodiment of the invention;
**FIGS. 2A-2C** schematically represent three illustrative embodiments of implants and/or implant components, in accordance with various embodiments of the invention;
**FIGS. 3A- 3B** depict designs of implant components that have six bone cuts (**FIG. 3A**), seven bone cuts (**FIG. 3B**), in accordance with various embodiments of the invention;

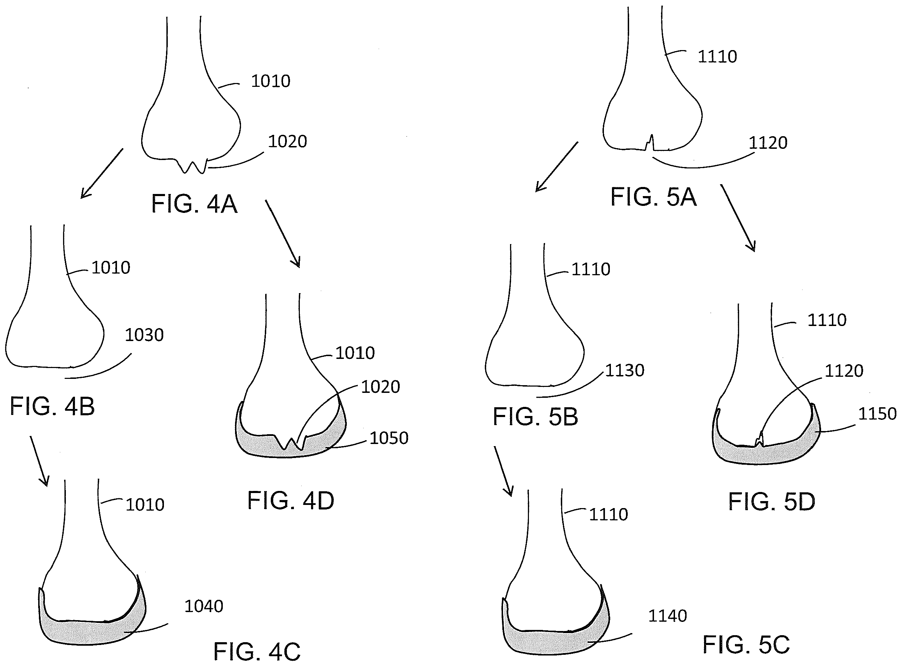

**FIG. 4A** is a drawing of a cross-sectional view of an end of a femur with an osteophyte; **FIG. 4B** is a drawing of the end of the femur of **FIG. 4A** with the osteophyte virtually removed; **FIG. 4C** is a drawing of the end of the femur of **FIG. 4B** with the osteophyte virtually removed and showing a cross-sectional view of an implant designed to the shape of the femur with the osteophyte removed; **FIG. 4D** is a drawing of the end of the femur of **FIG. 4A** and shows a cross-sectional view of an implant designed to the shape of the femur with the osteophyte intact, in accordance with various embodiments of the invention;
**FIG. 5A** is a drawing of a cross-sectional view of an end of a femur with a subchondral void in the bone; **FIG. 5B** is a drawing of the end of the femur of **FIG. 5A** with the void virtually removed; **FIG. 5C** is a drawing of the end of the femur of **FIG. 5B** with the void virtually removed and showing a cross-sectional view of an implant designed to the shape of the femur with the void removed; **FIG. 5D** is a drawing of the end of the femur of **FIG. 5A** and showing a cross-sectional view of an implant designed to the shape of the femur with the void intact, in accordance with various embodiments of the invention;
**FIG. 6** illustrates a coronal plane of the knee with exemplary resection cuts that can be used to correct lower limb alignment in a knee replacement;
**FIG. 7** depicts a coronal plane of the knee shown with femoral implant medial and lateral condyles having different thicknesses to help to correct limb alignment;
**FIGS. 8A-8C** illustrate a femoral implant component having six bones cuts that include one or more parallel and non-coplanar facets;
**FIGS. 9A-9B** illustrate a femoral implant component having seven bones cuts that include one or more parallel and non-coplanar facets;
**FIGS. 10A** and **10B** are schematic views of a femur and patella and exemplary resection cut planes;
**FIG. 11** is a schematic view of a sagittal plane of a femur with facet cuts indicated;
**FIG. 12** is a flow chart illustrating an exemplary process for selecting and/or designing a patient-adapted total knee implant, in accordance with an embodiment of the invention;
**FIG. 13A** illustrates a distal femur and a distal resection plane parallel to the epicondylar axis; **FIG. 13B** shows an example of an anterior oblique resection plane **8830.**
**FIGS. 14A** to **14E** show optimized resection cut planes to a patient's femur based on the medial condyle.
**FIGS. 15A** and 15**B** show resection cut planes for a patient's lateral condyle posterior chamfer (**FIG. 15A****)** and lateral condyle posterior (**FIG. 15B**) cut planes that are independently optimized based on patient-specific data for the lateral condyle;
**FIGS. 16A** and **16B** illustrate two exemplary distal resection cut planes for two different cut designs;
**FIGS. 17A** and 17**B** illustrate five femoral resection cuts for the two designs shown in **FIGS. 16A** and **16B****,** respectively;
**FIGS. 18A** and **18B** illustrate the completed cut femur models for each of two cut designs;
**FIG. 19A** illustrates an embodiment of a cut plane design having anterior and posterior cut planes that diverge from the component peg axis; **FIG. 19B** illustrates an implant component design that includes a peg diameter of 7 mm with a rounded tip, in accordance with an embodiment of the invention;
**FIGS. 20A** and **20B** illustrate exemplary bone-facing surfaces of femoral implant component designs that include a patient-adapted peripheral margin, in accordance with various embodiments of the invention;
**FIGS. 21A** and **21B** illustrate side views of exemplary femoral implant component designs, in accordance with an embodiment of the invention;
**FIG. 22** illustrates a femoral implant component (PCL-retaining) having seven bone cuts, in accordance with various embodiments of the invention;
**FIG. 23A** and **FIG. 23B** illustrate the resection cut planes for the implant component of FIG. 22;
**FIG. 24** illustrates the implant component of **FIG. 22** from a different angle to show cement pocket and peg features, in accordance with an embodiment of the invention;
**FIG. 25A** shows a five-cut-plane femoral resection design for a femoral implant component having five bone cuts; **FIG. 25B** shows a seven-cut-plane femoral resection design for a femoral implant component having seven bone cuts;
**FIG. 26** **A** shows a patient's femur having five, not flexed resection cuts; **FIG. 26B** shows the same femur but with five, flexed resection cuts;

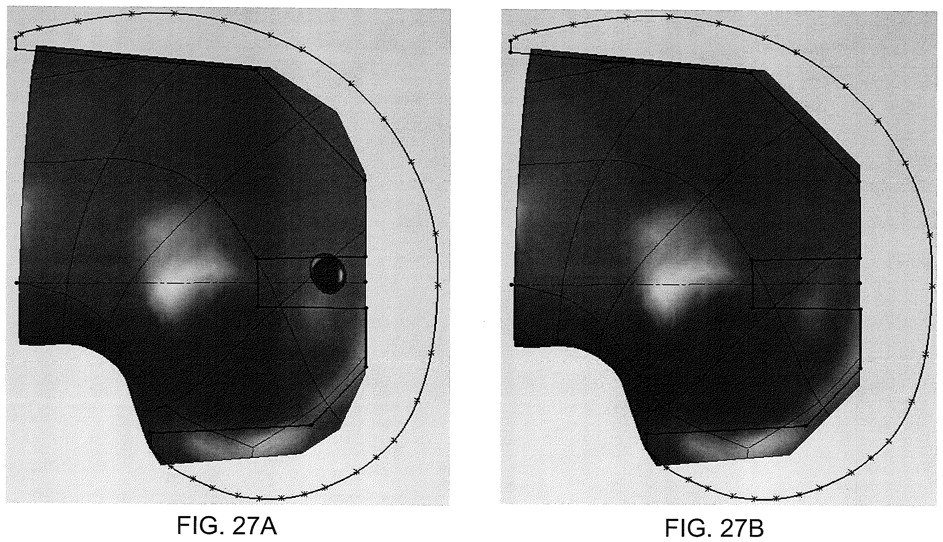

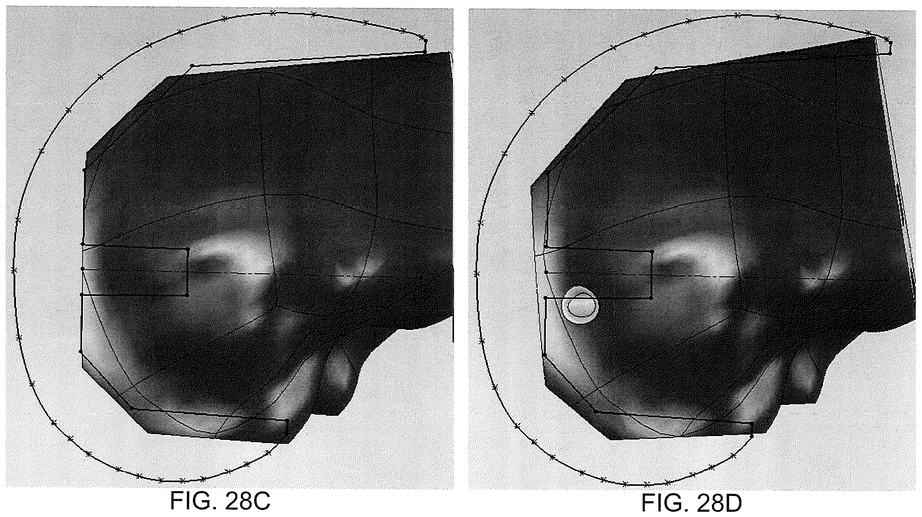

**FIGS. 27A** to **27D** show outlines of a traditional five-cut femoral component (in hatched lines) overlaid with, in **27A,** a femur having seven optimized resection cuts for matching an optimized seven-bone-cut implant component; in **FIG. 27B****,** a femur having five optimized resection cuts for matching to an optimized five-bone-cut implant component; in **FIG. 27C,** a femur having five, not flexed resection cuts for matching to an optimized five-bone-cut implant component; and in **FIG. 27D,** a femur having five, flexed resection cuts for matching to an optimized five-bone-cut, flexed implant component, in accordance with various embodiments of the invention;

Additional figure descriptions are included in the text below. Unless otherwise denoted in the description for each figure, "M" and "L" in certain figures indicate medial and lateral sides of the view; "A" and "P" in certain figures indicate anterior and posterior sides of the view, and "S" and "I" in certain figures indicate superior and inferior sides of the view.

### DETAILED DESCRIPTION

The invention is defined by the appended claims.

### Introduction

When a surgeon uses a traditional off-the-shelf implant to replace a patient's joint, for example, a knee joint, hip joint, or shoulder joint, certain features of the implant typically do not match the particular patient's biological features. These mismatches can cause various complications during and after surgery. For example, surgeons may need to extend the surgery time and apply estimates and rules of thumb during surgery to address the mismatches. For the patient, complications associated with these mismatches can include pain, discomfort, soft tissue impingement, and an unnatural feeling of the joint during motion, e.g., so-called mid-flexion instability, as well as an altered range of movement and an increased likelihood of implant failure. In order to fit a traditional implant component to a patient's articular bone, surgeons typically remove substantially more of the patient's bone than is necessary to merely clear diseased bone from the site. This removal of substantial portions of the patient's bone frequently diminishes the patient's bone stock to the point that only one subsequent revision implant is possible.

Certain embodiments of the implants, guide tools, and related methods of designing (e.g., designing and making), and using the implants and guide tools described herein can be applied to any joint including, without limitation, a spine, spinal articulations, an intervertebral disk, a facet joint, a shoulder, an elbow, a wrist, a hand, a finger, a hip, a knee, an ankle, a foot, or a toe joint. Furthermore, various embodiments described herein can apply to methods and procedures, and the design of methods and procedures, for resectioning the patient's anatomy in order to implant the implant components described herein and/or to using the guide tools described herein.

In certain embodiments, implant components and/or related methods described herein can include a combination of patient-specific and patient-engineered features. For example, patient-specific data collected preoperatively can be used to engineer one or more optimized surgical cuts to the patient's bone and to design or select a corresponding implant component having or more bone-facing surfaces or facets (i.e., "bone cuts") that specifically match one or more of the patient's resected bone surfaces. The surgical cuts to the patient's bone can be optimized (i.e., patient-engineered) to enhance one or more parameters, such as:
(1) deformity correction and limb alignment (2) maximizing preservation of bone, cartilage, or ligaments, or (3) restoration and/or optimization of joint kinematics or biomechanics. Based on the optimized surgical cuts and, optionally, on other desired features of the implant component, the implant component's bone-facing surface can be designed or selected to, at least in part, negatively-match the shape of the patient's resected bone surface.

### Improved implants, guide tools and related methods

Certain embodiments are directed to implants, guide tools, and/or related methods that can be used to provide to a patient a pre-primary procedure and/or a pre-primary implant such that a subsequent, replacement implant can be performed with a second (and, optionally, a third, and optionally, a fourth) patient-adapted pre-primary implant or with a traditional primary implant. In certain embodiments, the pre-primary implant procedure can include 3, 4, 5, 6, 7, or more resection or surgical cuts to the patient's bone and the pre-primary implant can include on its corresponding bone-facing surface a matching number and orientation of bone-cut facets or surfaces.

In one illustrative embodiment, a first pre-primary joint-replacement procedure includes a patient-adapted implant component, guide tool, and/or related method. The patient-adapted implant component, guide tool, and/or related method can be preoperatively selected and/or designed from patient-specific data, such as one or more images of the patient's joint, to include one or more features that are patient-specific or patient-engineered. The features (e.g., dimensions, shape, surface contours) of the first pre-primary implant and, optionally, patient-specific data (e.g., features of the patient's resected bone surfaces and features of the patient's contralateral joint) can be stored in a database. When the first pre-primary implant fails, for example, due to bone loss or osteolysis or aseptic loosening at a later point in time (e.g., 15 years after the original implantation) a second implant can be implanted. For the second implant procedure, the amount of diseased bone can be assessed. If the amount of diseased bone to be resected is minimal, the patient-specific data can be used to select and/or design a second pre-primary procedure and/or a pre-primary implant. If the amount of diseased bone to be resected is substantial, a traditional primary procedure and a traditional implant can be employed.

Alternatively, certain embodiments are directed to implants, guide tools, and/or related methods that can be used to provide to a patient a primary procedure and/or a primary implant such that a subsequent replacement implant can be used as part of a traditional revision procedure. Certain embodiments are directed to implants, guide tools, and/or related methods that can be used to provide a patient-adapted revision implant. For example, following a traditional implant, a subsequent revision can include a patient-adapted procedure and/or a patient-adapted implant component as described herein.

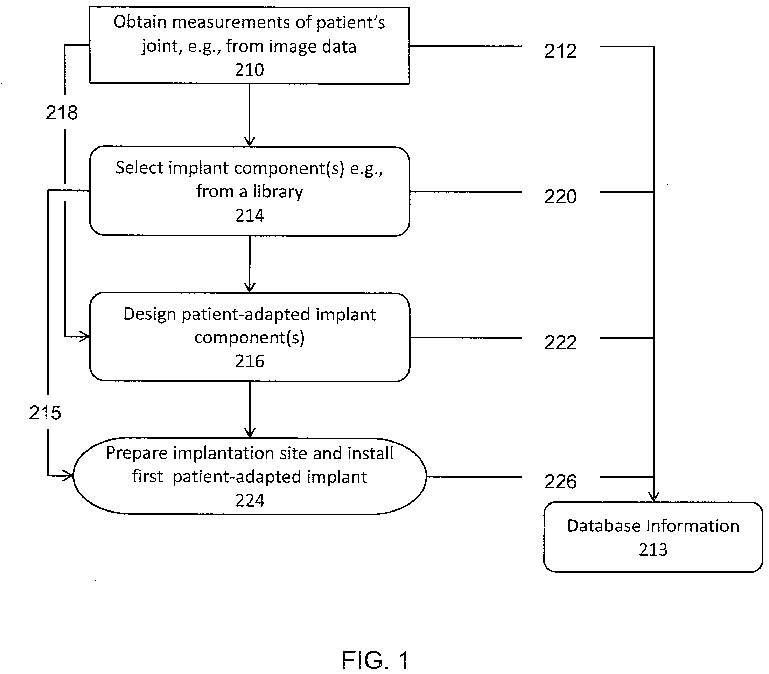

**FIG. 1** is a flow chart illustrating a process that includes selecting and/or designing a first patient-adapted implant, for example, a pre-primary implant. First, using the techniques described herein or those suitable and known in the art, measurements of the target joint are obtained **210.** This step can be repeated multiple times, as desired. Optionally, a virtual model of the joint can be generated, for example, to determine proper joint alignment and the corresponding resection cuts and implant component features based on the determined proper alignment. This information can be collected and stored **212** in a database **213.** Once measurements of the target joint are obtained and analyzed to determine resection cuts and patient-adapted implant features, the patient-adapted implant components can be selected **214** (e.g., selected from a virtual library and optionally manufactured without further design alteration **215,** or selected from a physical library of implant components). Alternatively, or in addition, one or more implant components with best-fitting and/or optimized features can be selected **214** (e.g., from a library) and then further designed (e.g., designed and manufactured) **216.** Alternatively or in addition, one or more implant components with best-fitting and/or optimized features can be designed (e.g., designed and manufactured) **218, 216** without an initial selection from a library. Using a virtual model to assess the selected or designed implant component(s), this process also can be repeated as desired (e.g., before one or more physical components are selected and/or generated). The information regarding the selected and/or designed implant component(s) can be collected and stored **220, 222** in a database **213.** Once a desired first patient-adapted implant component or set of implant components is obtained, a surgeon can prepare the implantation site and install the first implant **224.** The information regarding preparation of the implantation site and implant installation can be collected and stored **226** in a database **213.** In this way, the information associated with the first pre-primary implant component is available for use by a surgeon for subsequent implantation of a second pre-primary or a primary implant.

The term "implant component" as used herein can include: (i) one of two or more devices that work together in an implant or implant system, or (ii) a complete implant or implant system, for example, in embodiments in which an implant is a single, unitary device. The term "match" as used herein is envisioned to include one or both of a negative-match, as a convex surface fits a concave surface, and a positive-match, as one surface is identical to another surface.

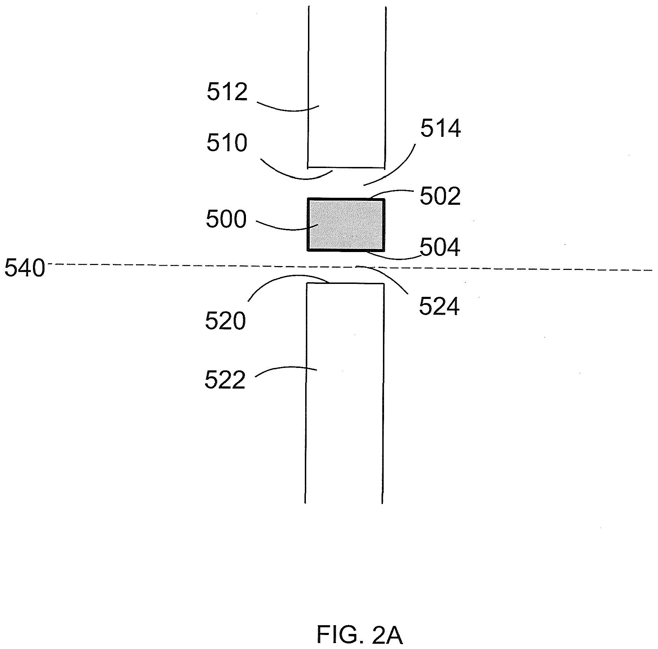

Three illustrative embodiments of implants and/or implant components are schematically represented in **FIGS. 2A-2C****.** In **FIG. 2A****,** the illustrative implant component **500** includes an inner, bone-facing surface **502** and an outer, joint-facing surface **504.** The inner bone-facing surface **502** engages a first articular surface **510** of a first biological structure **512,** such as bone or cartilage, at a first interface **514.** The articular surface **510** can be a native surface, a resected surface, or a combination of the two. The outer, joint-facing surface **504** opposes a second articular surface **520** of a second biological structure **522** at a joint interface **524.** The dashed line across each figure illustrates a patient's joint-line. In certain embodiments, one or more features of the implant component, for example, an M-L, A-P, or S-I dimension, a feature of the inner, bone-facing surface **502,** and/or a feature of the outer, joint-facing surface **504,** are patient-adapted (i.e., include one or more patient-specific and/or patient-engineered features).

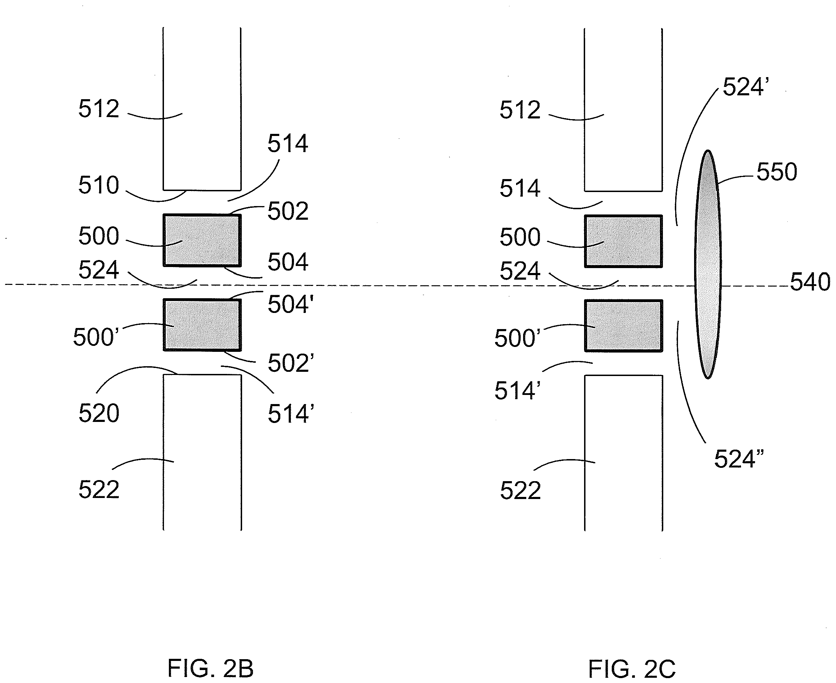

The illustrative embodiment shown in **FIG. 2B** includes two implant components **500, 500'.** Each implant component **500, 500'** includes an inner, bone-facing surface **502, 502'** and an outer, joint-facing surface **504, 504'.** The first inner, bone-facing surface **502** engages a first articular surface **510** of a first biological structure **512** (e.g., bone or cartilage) at a first interface **514.** The first articular surface **510** can be a native surface, a cut surface, or a combination of the two. The second bone-facing surface **502'** engages a second articular surface **520** of a second biological structure **522** at a second interface **514'.** The second articular surface **520** can be a native surface, a resected surface, or a combination of the two. In addition, an outer, joint-facing surface **504** on the first component **500** opposes a second, outer joint-facing surface **504'** on the second component **500'** at the joint interface **524.** In certain embodiments, one or more features of the implant component, for example, one or both of the inner, bone-facing surfaces **502, 502'** and/or one or both of the outer, joint-facing surfaces **504, 504',** are patient-adapted (i.e., include one or more patient-specific and/or patient-engineered features).

The illustrative embodiment represented in **FIG. 2C** includes the two implant components **500, 500',** the two biological structures **512, 522,** the two interfaces **514, 514',** and the joint interface **524,** as well as the corresponding surfaces, as described for the embodiment illustrated in **FIG. 2B****.** However, **FIG. 2C** also includes structure **550,** which can be an implant component in certain embodiments or a biological structure in certain embodiments. Accordingly, the presence of a third structural **550** surface in the joint creates a second joint interface **524',** and possibly a third **524",** in addition to joint interface **524.** Alternatively or in addition to the patient-adapted features described above for components **500** and **500',** the components **500, 500'** can include one or more features, such as surface features at the additional joint interface(s) **524, 524",** as well as other dimensions (e.g., height, width, depth, contours, and other dimensions) that are patient-adapted, in whole or in part. Moreover, structure **550,** when it is an implant component, also can have one or more patient-adapted features, such as one or more patient-adapted surfaces and dimensions.

Traditional implants and implant components can have surfaces and dimensions that are a poor match to a particular patient's biological feature(s). The patient-adapted implants, guide tools, and related methods described herein improve upon these deficiencies. The following two subsections describe two particular improvements, with respect to the bone-facing surface and the joint-facing surface of an implant component; however, the principles described herein are applicable to any aspect of an implant component.

### Bone-facing surface of an implant component

In certain embodiments, the bone-facing surface of an implant can be designed to substantially negatively-match one more bone surfaces. For example, in certain embodiments at least a portion of the bone-facing surface of a patient-adapted implant component can be designed to substantially negatively-match the shape of subchondral bone, cortical bone, endosteal bone, and/or bone marrow. A portion of the implant also can be designed for resurfacing, for example, by negatively-matching portions of a bone-facing surface of the implant component to the subchondral bone or cartilage. Accordingly, in certain embodiments, the bone-facing surface of an implant component can include one or more portions designed to engage resurfaced bone, for example, by having a surface that negatively-matches uncut subchondral bone or cartilage, and one or more portions designed to engage cut bone, for example, by having a surface that negatively-matches a cut subchondral bone.

In certain embodiments, the bone-facing surface of an implant component includes multiple surfaces, also referred to herein as bone cuts. One or more of the bone cuts on the bone-facing surface of the implant component can be selected and/or designed to substantially negatively-match one or more surfaces of the patient's bone. The surface(s) of the patient's bone can include bone, cartilage, or other biological surfaces. For example, in certain embodiments, one or more of the bone cuts on the bone-facing surface of the implant component can be designed to substantially negatively-match (e.g., the number, depth, and/or angles of cut) one or more resected surfaces of the patient's bone. The bone-facing surface of the implant component can include any number of bone cuts, for example, two, three, four, less than five, five, more than five, six, seven, eight, nine or more bone cuts. In certain embodiments, the bone cuts of the implant component and/or the resection cuts to the patient's bone can include one or more facets on corresponding portions of an implant component. For example, the facets can be separated by a space or by a step cut connecting two corresponding facets that reside on parallel or non-parallel planes. These bone-facing surface features can be applied to various joint implants, including knee, hip, spine, and shoulder joint implants.

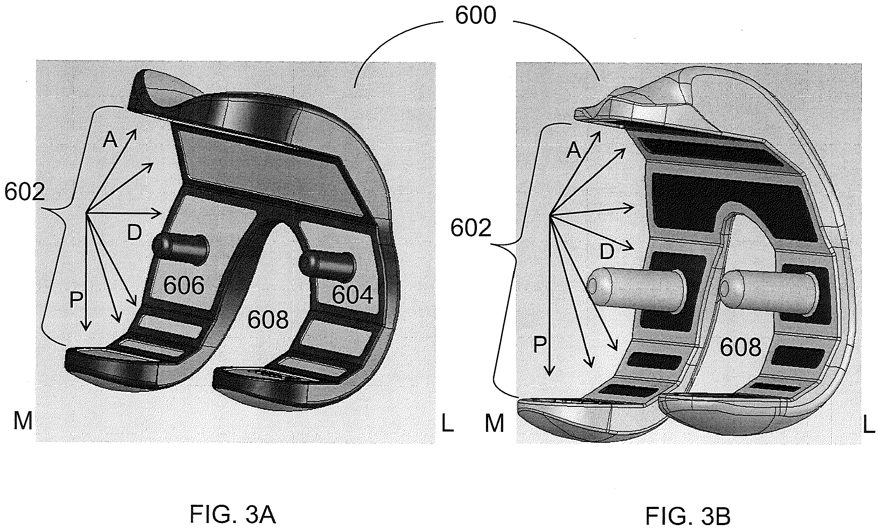

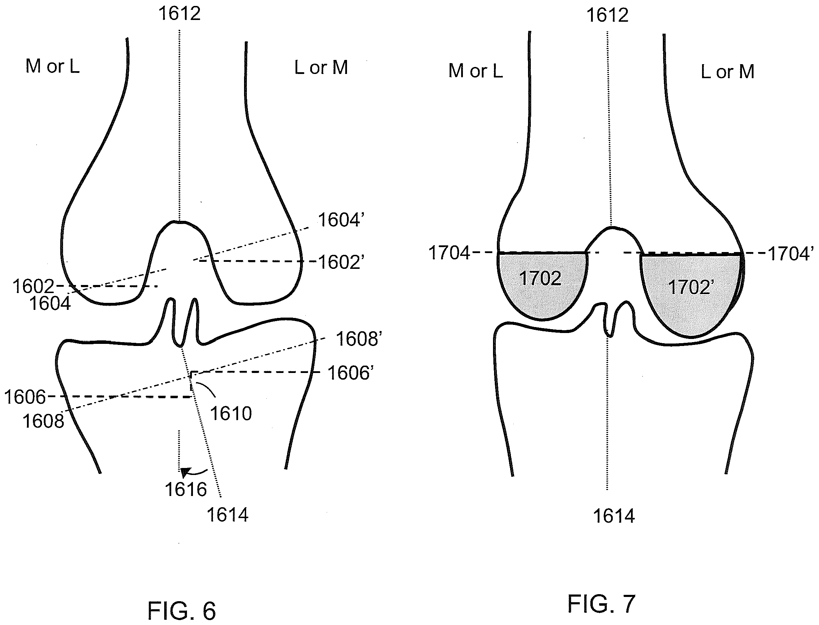

**FIG. 3A** illustrates an exemplary femoral implant component **600** having six bone cuts. **FIG. 3B** illustrates a femoral implant component **600** having seven bone cuts. In **FIG. 3A** and **FIG. 3B****,** the six or seven respective bone cuts are identified by arrows on the inner, bone-facing surface **602** of the implant component **600.** The bone cuts can include, for example, an anterior bone cut **A,** a distal bone cut **D,** and a posterior bone cut **P,** as well as one or more anterior chamfer bone cuts between the anterior bone cut **A** and distal bone cut **D,** and/or one or more posterior chamfer bone cuts between the distal posterior bone cut **P** and the distal bone cut **D.** The implant component depicted in **FIG. 6A** includes one anterior chamfer bone cut and two posterior chamfer bone cuts, in addition to anterior, posterior and distal bone cuts. The implant component depicted in **FIG. 6B** includes two anterior chamfer bone cuts and two posterior chamfer bone cuts, in addition to anterior, posterior and distal bone cuts.

Any one or more bone cuts can include one or more facets. For example, the implant components exemplified in **FIG. 3A** and **FIG. 3B** depict corresponding condylar facets for each of the distal bone cut, posterior bone cut, first posterior chamfer bone cut and second posterior chamfer bone cut. In **FIG. 3A****,** distal bone cut facets on lateral and medial condyles are identified by **604** and **606,** respectively. Facets of a bone cut can be separated by a space between corresponding regions of an implant component, as exemplified by the condylar facets separated by the intercondylar space **608** in **FIG. 3A** and **FIG. 3B****.** Alternatively or in addition, facets of a bone cut can be separated by a step cut, for example, a vertical or angled cut connecting two non-coplanar or non facets of a bone cut. As shown by the implant components exemplified in each of **FIG. 3A** and **FIG. 3B****,** each bone cut and/or bone cut facet can be substantially planar.

In certain embodiments, corresponding sections of an implant component can include different thicknesses (i.e., distance between the component's bone-facing surface and joint-facing surface), surface features, bone cut features, section volumes, and/or other features. For example, as shown in **FIG. 3A****,** the corresponding distal lateral and medial sections of the implant, identified by **604** and **606** on their respective bone cut facets, include different thicknesses, section volumes, bone cut angles, and bone cut surface areas. As this example illustrates, one or more of the thicknesses, section volumes, bone cut angles, bone cut surface areas, bone cut curvatures, numbers of bone cuts, peg placements, peg angles, and other features may vary between two or more sections (e.g., corresponding sections on lateral and medial condyles) of an implant component. Alternatively or in addition, one, more, or all of these features can be the same in corresponding sections of an implant component. An implant design that allows for independent features on different sections of an implant allows various options for achieving one or more goals, including, for example, (1) deformity correction and limb alignment (2) preserving bone, cartilage, and/or ligaments.

Alternatively or in addition, one or more aspects of an implant component, for example, one or more bone cuts, can be selected and/or designed to match predetermined resection cuts. Predetermined as used herein includes, for example, preoperatively determined (e.g., preoperatively selected and/or designed). For example, predetermined resection cuts can include resection cuts determined preoperatively, optionally as part of the selection and/or design of one or more implant components and/or one or more guide tools. Similarly, a surgical guide tool can be selected and/or designed to guide the predetermined resection cuts. For example, the resection cuts and matching component bone cuts (and, optionally, a guide tool) can be selected and/or designed, for example, to remove diseased or malformed tissue and/or to optimize a desired anatomical and/or kinematic parameter, such as maximizing bone preservation, correcting a joint and/or alignment deformity, enhancing joint kinematics, enhancing or preserving joint-line location, and/or other parameter(s) described herein.

### Joint-facing surface of an implant component

In various embodiments described herein, the outer, joint-facing surface of an implant component includes one or more patient-adapted (e.g., patient-specific and/or patient-engineered features). For example, in certain embodiments, the joint-facing surface of an implant component can be designed to match the shape of the patient's biological structure. The joint-facing surface can include, for example, the bearing surface portion of the implant component that engages an opposing biological structure or implant component in the joint to facilitate typical movement of the joint. The patient's biological structure can include, for example, cartilage, bone, and/or one or more other biological structures.

For example, in certain embodiments, the joint-facing surface of an implant component is designed to match the shape of the patient's articular cartilage. For example, the joint-facing surface can substantially positively-match one or more features of the patient's existing cartilage surface and/or healthy cartilage surface and/or a calculated cartilage surface, on the articular surface that the component replaces. Alternatively, it can substantially negatively-match one or more features of the patient's existing cartilage surface and/or healthy cartilage surface and/or a calculated cartilage surface, on the opposing articular surface in the joint. As described below, corrections can be performed to the shape of diseased cartilage by designing surgical steps (and, optionally, patient-adapted surgical tools) to re-establish a normal or near normal cartilage shape that can then be incorporated into the shape of the joint-facing surface of the component. These corrections can be implemented and, optionally, tested in virtual two-dimensional and three-dimensional models. The corrections and testing can include kinematic analysis and/or surgical steps.

In certain embodiments, the joint-facing surface of an implant component can be designed to positively-match the shape of subchondral bone. For example, the joint-facing surface of an implant component can substantially positively-match one or more features of the patient's existing subchondral bone surface and/or healthy subchondral bone surface and/or a calculated subchondral bone surface, on the articular surface that the component attaches to on its bone-facing surface. Alternatively, it can substantially negatively-match one or more features of the patient's existing subchondral bone surface and/or healthy subchondral bone surface and/or a calculated subchondral bone surface, on the opposing articular surface in the joint. Corrections can be performed to the shape of subchondral bone to re-establish a normal or near normal articular shape that can be incorporated into the shape of the component's joint-facing surface. A standard thickness can be added to the joint-facing surface, for example, to reflect an average cartilage thickness. Alternatively, a variable thickness can be applied to the component. The variable thickness can be selected to reflect a patient's actual or healthy cartilage thickness, for example, as measured in the individual patient or selected from a standard reference database.

In certain embodiments, the joint-facing surface of an implant component can include one or more standard features. The standard shape of the joint-facing surface of the component can reflect, at least in part, the shape of typical healthy subchondral bone or cartilage. For example, the joint-facing surface of an implant component can include a curvature having standard radii or curvature in one or more directions. Alternatively or in addition, an implant component can have a standard thickness or a standard minimum thickness in select areas. Standard thickness(es) can be added to one or more sections of the joint-facing surface of the component or, alternatively, a variable thickness can be applied to the implant component.

Certain embodiments, such as those illustrated by **FIG. 2B** and 2**C**, include, in addition to a first implant component, a second implant component having an opposing joint-facing surface. The second implant component's bone-facing surface and/or joint-facing surface can be designed as described above. Moreover, in certain embodiments, the joint-facing surface of the second component can be designed, at least in part, to match (e.g., substantially negatively-match) the joint-facing surface of the first component. Designing the joint-facing surface of the second component to complement the joint-facing surface of the first component can help reduce implant wear and optimize kinematics. Thus, in certain embodiments, the joint-facing surfaces of the first and second implant components can include features that do not match the patient's existing anatomy, but instead negatively-match or nearly negatively-match the joint-facing surface of the opposing implant component.

However, when a first implant component's joint-facing surface includes a feature adapted to a patient's biological feature, a second implant component having a feature designed to match that feature of the first implant component also is adapted to the patient's same biological feature. By way of illustration, when a joint-facing surface of a first component is adapted to a portion of the patient's cartilage shape, the opposing joint-facing surface of the second component designed to match that feature of the first implant component also is adapted to the patient's cartilage shape. When the joint-facing surface of the first component is adapted to a portion of a patient's subchondral bone shape, the opposing joint-facing surface of the second component designed to match that feature of the first implant component also is adapted to the patient's subchondral bone shape. When the joint-facing surface of the first component is adapted to a portion of a patient's cortical bone, the joint-facing surface of the second component designed to match that feature of the first implant component also is adapted to the patient's cortical bone shape. When the joint-facing surface of the first component is adapted to a portion of a patient's endosteal bone shape, the opposing joint-facing surface of the second component designed to match that feature of the first implant component also is adapted to the patient's endosteal bone shape. When the joint-facing surface of the first component is adapted to a portion of a patient's bone marrow shape, the opposing joint-facing surface of the second component designed to match that feature of the first implant component also is adapted to the patient's bone marrow shape.

The opposing joint-facing surface of a second component can substantially negatively-match the joint-facing surface of the first component in one plane or dimension, in two planes or dimensions, in three planes or dimensions, or in several planes or dimensions. For example, the opposing joint-facing surface of the second component can substantially negatively-match the joint-facing surface of the first component in the coronal plane only, in the sagittal plane only, or in both the coronal and sagittal planes.

In creating a substantially negatively-matching contour on an opposing joint-facing surface of a second component, geometric considerations can improve wear between the first and second components. For example, the radii of a concave curvature on the opposing joint-facing surface of the second component can be selected to match or to be slightly larger in one or more dimensions than the radii of a convex curvature on the joint-facing surface of the first component. Similarly, the radii of a convex curvature on the opposing joint-facing surface of the second component can be selected to match or to be slightly smaller in one or more dimensions than the radii of a concave curvature on the joint-facing surface of the first component. In this way, contact surface area can be maximized between articulating convex and concave curvatures on the respective surfaces of first and second implant components.

The bone-facing surface of the second component can be designed to negatively-match, at least in part, the shape of articular cartilage, subchondral bone, cortical bone, endosteal bone or bone marrow (e.g., surface contour, angle, or perimeter shape of a resected or native biological structure). It can have any of the features described above for the bone-facing surface of the first component, such as having one or more patient-adapted bone cuts to match one or more predetermined resection cuts.

Many combinations of first component and second component bone-facing surfaces and joint-facing surfaces are possible. **Table 1** provides illustrative combinations that may be employed.

**Table 1: Illustrative Combinations of Implant Components**

| **1^{st} component bone-facing surface** | **1^{st} component joint-facing surface** | **1^{st} component bone cut(s)** | **2^{nd} component joint facing surface** | **2^{nd} component bone facing surface** | **2^{nd} component bone cuts** |
|---|---|---|---|---|---|
| **Example: Femur** | Example: Femur | Example: Femur | Example: Tibia | Example: Tibia | Example: Tibia |
| **At least one bone cut** | Cartilage | Yes | Negative-match of 1^{st} component joint-facing (opposing cartilage) | At least one bone cut | Yes |
| **At least one bone cut** | Cartilage | Yes | Negative-match of 1^{st} component joint-facing (opposing cartilage) | Subchondral bone | Optional |
| **At least one bone cut** | Cartilage | Yes | Negative-match of 1^{st} component joint-facing (opposing cartilage) | Cartilage (same side, e.g. tibia) | Optional |
| **At least one bone cut** | Subchondral bone | Yes | Negative-match of 1^{st} component joint-facing (opposing subchondral bone) | At least one bone cut | Yes |
| **At least one bone cut** | Subchondral bone | Yes | Negative-match of 1^{st} component joint-facing (opposing subchondral bone) | Subchondral bone | Optional |
| **At least one bone cut** | Subchondral bone | Yes | Negative-match of 1^{st} component joint-facing (opposing subchondral bone) | Cartilage (same side, e.g. tibia) | Optional |
| **Subchondral bone** | Cartilage | Optional | Negative-match of 1^{st} component joint-facing (opposing cartilage) | At least one bone cut | Yes |
| **Subchondral bone** | Cartilage | Optional | Negative-match of 1^{st} component joint-facing (opposing cartilage) | Subchondral bone | Optional |
| **Subchondral bone** | Cartilage | Optional | Negative-match of 1^{st} component joint-facing (opposing cartilage) | Cartilage (same side, e.g. tibia) | Optional |
| **Subchondral bone** | Subchondral bone | Optional | Negative-match of 1^{st} component joint-facing (opposing subchondral bone) | At least one bone cut | Yes |
| **Subchondral bone** | Subchondral bone | Optional | Negative-match of 1^{st} component joint-facing (opposing subchondral bone) | Subchondral bone | Optional |
| **Subchondral bone** | Subchondral bone | Optional | Negative-match of 1^{st} component joint-facing (opposing subchondral bone) | Cartilage (same side, e.g. tibia) | Optional |
| **Subchondral bone** | Standard / Model | Optional | Negative-match of 1^{st} component joint-facing standard | At least one bone cut | Yes |
| **Subchondral bone** | Standard / Model | Optional | Negative-match of 1^{st} component joint-facing standard | Subchondral bone | Optional |
| **Subchondral bone** | Standard / Model | Optional | Negative-match of 1^{st} component joint-facing standard | Cartilage (same side, e.g. tibia) | Optional |
| **Subchondral bone** | Subchondral bone | Optional | Non-matching standard surface | At least one bone cut | Yes |
| **Subchondral bone** | Cartilage | Optional | Non-matching standard surface | At least one bone cut | Yes |

Embodiments described herein can be applied to partial or total joint replacement systems. Bone cuts or changes to an implant component dimension described herein can be applied to a portion of the dimension, or to the entire dimension.

### Collecting and modeling patient-specific data

As mentioned above, certain embodiments include implant components designed and made using patient-specific data that is collected preoperatively. The patient-specific data can include points, surfaces, and/or landmarks, collectively referred to herein as "reference points." In certain embodiments, the reference points can be selected and used to derive a varied or altered surface, such as, without limitation, an ideal surface or structure. For example, the reference points can be used to create a model of the patient's relevant biological feature(s) and/or one or more patient-adapted surgical steps, tools, and implant components. For example the reference points can be used to design a patient-adapted implant component having at least one patient-specific or patient-engineered feature, such as a surface, dimension, or other feature.

Sets of reference points can be grouped to form reference structures used to create a model of a joint and/or an implant design. Designed implant surfaces can be derived from single reference points, triangles, polygons, or more complex surfaces or models of joint material, such as, for example, articular cartilage, subchondral bone, cortical bone, endosteal bone or bone marrow. Various reference points and reference structures can be selected and manipulated to derive a varied or altered surface, such as, without limitation, an ideal surface or structure.

The reference points can be located on or in the joint that will receive the patient-specific implant. For example, the reference points can include weight-bearing surfaces or locations in or on the joint, a cortex in the joint, and/or an endosteal surface of the joint. The reference points also can include surfaces or locations outside of but related to the joint. Specifically, reference points can include surfaces or locations functionally related to the joint. For example, in embodiments directed to the knee joint, reference points can include one or more locations ranging from the hip down to the ankle or foot. The reference points also can include surfaces or locations homologous to the joint receiving the implant. For example, in embodiments directed to a knee, a hip, or a shoulder joint, reference points can include one or more surfaces or locations from the contralateral knee, hip, or shoulder joint.

In certain embodiments, an imaging data collected from the patient, for example, imaging data from one or more of x-ray imaging, digital tomosynthesis, cone beam CT, non-spiral or spiral CT, non-isotropic or isotropic MRI, SPECT, PET, ultrasound, laser imaging, photo-acoustic imaging, is used to qualitatively and/or quantitatively measure one or more of a patient's biological features, one or more of normal cartilage, diseased cartilage, a cartilage defect, an area of denuded cartilage, subchondral bone, cortical bone, endosteal bone, bone marrow, a ligament, a ligament attachment or origin, menisci, labrum, a joint capsule, articular structures, and/or voids or spaces between or within any of these structures.

In certain embodiments, the model that includes at least a portion of the patient's joint also can include or display, as part of the model, one or more resection cuts, one or more drill holes, (e.g., on a model of the patient's femur), one or more guide tools, and/or one or more implant components that have been designed for the particular patient using the model. Moreover, one or more resection cuts, one or more drill holes, one or more guide tools, and/or one or more implant components can be modeled and selected and/or designed separate from a model of a particular patient's biological feature.

### Modeling and addressing joint defects

In certain embodiments, the reference points and/or measurements described above can be processed using mathematical functions to derive virtual, corrected features, which may represent a restored, ideal or desired feature from which a patient-adapted implant component can be designed. For example, one or more features, such as surfaces or dimensions of a biological structure can be modeled, altered, added to, changed, deformed, eliminated, corrected and/or otherwise manipulated (collectively referred to herein as "variation" of an existing surface or structure within the joint).

Variation of the joint or portions of the joint can include, without limitation, variation of one or more external surfaces, internal surfaces, joint-facing surfaces, uncut surfaces, cut surfaces, altered surfaces, and/or partial surfaces as well as osteophytes, subchondral cysts, geodes or areas of eburnation, joint flattening, contour irregularity, and loss of normal shape. The surface or structure can be or reflect any surface or structure in the joint, including, without limitation, bone surfaces, ridges, plateaus, cartilage surfaces, ligament surfaces, or other surfaces or structures. The surface or structure derived can be an approximation of a healthy joint surface or structure or can be another variation. The surface or structure can be made to include pathological alterations of the joint. The surface or structure also can be made whereby the pathological joint changes are virtually removed in whole or in part.

Once one or more reference points, measurements, structures, surfaces, models, or combinations thereof have been selected or derived, the resultant shape can be varied, deformed or corrected. In certain embodiments, the variation can be used to select and/or design an implant component having an ideal or optimized feature or shape, e.g., corresponding to the deformed or corrected joint feature or shape. For example, in one application of this embodiment, the ideal or optimized implant shape reflects the shape of the patient's joint before he or she developed arthritis.

Alternatively or in addition, the variation can be used to select and/or design a patient-adapted surgical procedure to address the deformity or abnormality. For example, the variation can include surgical alterations to the joint, such as virtual resection cuts, virtual drill holes, virtual removal of osteophytes, and/or virtual building of structural support in the joint deemed necessary or beneficial to a desired final outcome for a patient.

### Osteophytes, subchondral voids, and other patient-specific defects

Corrections can be used to address osteophytes, subchondral voids, and other patient-specific defects or abnormalities. In the case of osteophytes, a design for the bone-facing surface of an implant component or guide tool can be selected and/or designed after the osteophyte has been virtually removed. Alternatively, the osteophyte can be integrated into the shape of the bone-facing surface of the implant component or guide tool. **FIGS. 4A-4D** are exemplary drawings of an end of a femur **1010** having an osteophyte **1020.** In the selection and/or design of an implant component for a particular patient, an image or model of the patient's bone that includes the osteophyte can be transformed such that the osteophyte **1020** is virtually removed, as shown in **FIG. 4B** at removed osteophyte **1030,** to produce, as shown in **FIG. 4C****,** an implant component **1040** based on a smooth surface at the end of femur **1010.** Alternatively, as shown in **FIG. 4D****,** an implant component **1050** can be selected and/or designed to conform to the shape of the osteophyte **1020.** In the case of building additional or improved structure, additional features of the implant component then can be derived after bone-facing surface correction is modeled. Optionally, a surgical strategy and/or one or more guide tools can be selected and/or designed to reflect the correction and correspond to the implant component.

Similarly, to address a subchondral void, a selection and/or design for the bone-facing surface of an implant component can be derived after the void has been virtually removed (e.g., filled). Alternatively, the subchondral void can be integrated into the shape of the bone-facing surface of the implant component. **FIGS. 5A-5D** are exemplary drawings of an end of a femur **1110** having a subchondral void **1120.** During development of an implant, an image or model of the patient's bone that includes the void can be transformed such that the void **1120** is virtually removed, as shown in **FIG. 5B** at removed void **1130,** to produce, as shown in **FIG. 5C****,** an implant component **1140** based on a smooth surface at the end of femur **1110.** Alternatively, implant **1110** can be selected and/or designed to conform to the shape of void **1120,** as shown in **FIG. 5D****.** Note that, while virtually conforming to void **1120,** implant **1150** may not practically be able to be inserted into the void. Therefore, in an certain embodiments, the implant may only partially protrude into a void in the bone. Optionally, a surgical strategy and/or one or more guide tools can be selected and/or designed to reflect the correction and correspond to the implant component.

Certain embodiments described herein include collecting and using data from imaging tests to virtually determine in one or more planes one or more of an anatomic axis and a mechanical axis and the related misalignment of a patient's limb. The imaging tests that can be used to virtually determine a patient's axis and misalignment can include one or more of such as x-ray imaging, digital tomosynthesis, cone beam CT, non-spiral or spiral CT, non-isotropic or isotropic MRI, SPECT, PET, ultrasound, laser imaging, and photoacoustic imaging, including studies utilizing contrast agents. Data from these tests can be used to determine anatomic reference points or limb alignment, including alignment angles within the same and between different joints or to simulate normal limb alignment. Using the image data, one or more mechanical or anatomical axes, angles, planes or combinations thereof can be determined. In certain embodiments, such axes, angles, and/or planes can include, or be derived from, one or more of a Whiteside's line, Blumensaat's line, transepicondylar line, femoral shaft axis, femoral neck axis, acetabular angle, lines tangent to the superior and inferior acetabular margin, lines tangent to the anterior or posterior acetabular margin, femoral shaft axis, tibial shaft axis, transmalleolar axis, posterior condylar line, tangent(s) to the trochlea of the knee joint, tangents to the medial or lateral patellar facet, lines tangent or perpendicular to the medial and lateral posterior condyles, lines tangent or perpendicular to a central weight-bearing zone of the medial and lateral femoral condyles, lines transecting the medial and lateral posterior condyles, for example through their respective centerpoints, lines tangent or perpendicular to the tibial tuberosity, lines vertical or at an angle to any of the aforementioned lines, and/or lines tangent to or intersecting the cortical bone of any bone adjacent to or enclosed in a joint. Moreover, estimating a mechanical axis, an angle, or plane also can be performed using image data obtained through two or more joints, such as the knee and ankle joint, for example, by using the femoral shaft axis and a centerpoint or other point in the ankle, such as a point between the malleoli.

As one example, if surgery of the knee or hip is contemplated, the imaging test can include acquiring data through at least one of, or several of, a hip joint, knee joint or ankle joint. As another example, if surgery of the knee joint is contemplated, a mechanical axis can be determined. For example, the centerpoint of the hip knee and ankle can be determined. By connecting the centerpoint of the hip with that of the ankle, a mechanical axis can be determined in the coronal plane. The position of the knee relative to said mechanical axis can be a reflection of the degree of varus or valgus deformity. The same determinations can be made in the sagittal plane, for example to determine the degree of *genu antecurvatum* or *recurvatum.* Similarly, any of these determinations can be made in any other desired planes, in two or three dimensions.

### Modeling articular cartilage

Cartilage loss in one compartment can lead to progressive joint deformity. For example, cartilage loss in a medial compartment of the knee can lead to varus deformity. In certain embodiments, cartilage loss can be estimated in the affected compartments. The estimation of cartilage loss can be done using an ultrasound MRI or CT scan or other imaging modality, optionally with intravenous or intra-articular contrast. The estimation of cartilage loss can be as simple as measuring or estimating the amount of joint space loss seen on x-rays. For the latter, typically standing x-rays are preferred. If cartilage loss is measured from x-rays using joint space loss, cartilage loss on one or two opposing articular surfaces can be estimated by, for example, dividing the measured or estimated joint space loss by two to reflect the cartilage loss on one articular surface. Other ratios or calculations are applicable depending on the joint or the location within the joint. Subsequently, a normal cartilage thickness can be virtually established on one or more articular surfaces by simulating normal cartilage thickness. In this manner, a normal or near normal cartilage surface can be derived. Normal cartilage thickness can be virtually simulated using a computer, for example, based on computer models, for example using the thickness of adjacent normal cartilage, cartilage in a contralateral joint, or other anatomic information including subchondral bone shape or other articular geometries. Cartilage models and estimates of cartilage thickness can also be derived from anatomic reference databases that can be matched, for example, to a patient's weight, sex, height, race, gender, or articular geometry(ies).

In certain embodiments, a patient's limb alignment can be virtually corrected by realigning the knee after establishing a normal cartilage thickness or shape in the affected compartment by moving the joint bodies, for example, femur and tibia, so that the opposing cartilage surfaces including any augmented or derived or virtual cartilage surface touch each other, typically in the preferred contact areas. These contact areas can be simulated for various degrees of flexion or extension.

### Deformity correction and optimizing limb alignment

Information regarding the misalignment and the proper mechanical alignment of a patient's limb can be used to preoperatively design and/or select one or more features of a joint implant and/or implant procedure. For example, based on the difference between the patient's misalignment and the proper mechanical axis, a knee implant and implant procedure can be designed and/or selected preoperatively to include implant and/or resection dimensions that substantially realign the patient's limb to correct or improve a patient's alignment deformity. In addition, the process can include selecting and/or designing one or more surgical tools (e.g., guide tools or cutting jigs) to direct the clinician in resectioning the patient's bone in accordance with the preoperatively designed and/or selected resection dimensions.

In certain embodiments, the degree of deformity correction that is necessary to establish a desired limb alignment is calculated based on information from the alignment of a virtual model of a patient's limb. The virtual model can be generated from patient-specific data, such 2D and/or 3D imaging data of the patient's limb. The deformity correction can correct varus or valgus alignment or antecurvatum or recurvatum alignment. In a preferred embodiment, the desired deformity correction returns the leg to normal alignment, for example, a zero degree biomechanical axis in the coronal plane and absence of genu antecurvatum and recurvatum in the sagittal plane.

**FIG. 6** illustrates a coronal plane of the knee with exemplary resection cuts that can be used to correct lower limb alignment in a knee replacement. As shown in the figure, the selected and/or designed resection cuts can include different cuts on different portions of a patient's biological structure. For example, resection cut facets on medial and lateral femoral condyles can be non-coplanar and parallel **1602, 1602',** angled **1604, 1604',** or non-coplanar and non-parallel, for example, cuts **1602** and **1604'** or cuts **1602'and 1604.** Similar, resection cut facets on medial and lateral portions of the tibia can be non-coplanar and parallel **1606**, **1606'**, angled and parallel **1608, 1608',** or non-coplanar and non-parallel, for example, cuts **1606** and **1608'** or cuts **1606'** and **1608.** Non-coplanar facets of resection cuts can include a step-cut **1610** to connect the non-coplanar resection facet surfaces. Selected and/or designed resection dimensions can be achieved using or more selected and/or designed guide tools (e.g., cutting jigs) that guide resectioning (e.g., guide cutting tools) of the patient's biological structure to yield the predetermined resection surface dimensions (e.g., resection surface(s), angles, and/or orientation(s). In certain embodiments, the bone-facing surfaces of the implant components can be designed to include one or more features (e.g., bone cut surface areas, perimeters, angles, and/or orientations) that substantially match one or more of the resection cut or cut facets that were predetermined to enhance the patient's alignment. As shown in **FIG. 6****,** certain combinations of resection cuts can aid in bringing the femoral mechanical axis **1612** and tibial mechanical axis **1614** into alignment **1616.**

Alternatively, or in addition, certain implant features, such as different implant thicknesses and/or surface curvatures across two different sides of the plane in which the mechanical axes **1612, 1614** are misaligned also can aid correcting limb alignment. For example, **FIG. 7** depicts a coronal plane of the knee shown with femoral implant medial and lateral condyles **1702, 1702'** having different thicknesses to help to correct limb alignment. These features can be used in combination with any of the resection cut **1704, 1704'** described above and/or in combination with different thicknesses on the corresponding portions of the tibial component. As described more fully below, independent tibial implant components and/or independent tibial inserts on medial and lateral sides of the tibial implant component can be used enhance alignment at a patient's knee joint. An implant component can include constant yet different thicknesses in two or more portions of the implant (e.g., a constant medial condyle thickness different from a constant lateral condyle thickness), a gradually increasing thickness across the implant or a portion of the implant, or a combination of constant and gradually increasing thicknesses.

### Preserving bone, cartilage or ligament

Traditional orthopedic implants incorporate bone cuts. These bone cuts achieve two objectives: they establish a shape of the bone that is adapted to the implant and they help achieve a normal or near normal axis alignment. For example, bone cuts can be used with a knee implant to correct an underlying varus of valgus deformity and to shape the articular surface of the bone to fit a standard, bone-facing surface of a traditional implant component. With a traditional implant, multiple bone cuts are placed. However, since traditional implants are manufactured off-the-shelf without use of patient-specific information, these bone cuts are pre-set for a given implant without taking into consideration the unique shape of the patient. Thus, by cutting the patient's bone to fit the traditional implant, more bone is discarded than is necessary with an implant designed to address the particularly patient's structures and deficiencies.

### Planning resection cuts for one or more articular surfaces

In certain embodiments, resection cuts are optimized to preserve the maximum amount of bone for each individual patient, based on a series of two-dimensional images or a three-dimensional representation of the patient's articular anatomy and geometry and the desired limb alignment and/or desired deformity correction. Resection cuts on two opposing articular surfaces can be optimized to achieve the minimum amount of bone resected from one or both articular surfaces.

By adapting resection cuts in the series of two-dimensional images or the three-dimensional representation on two opposing articular surfaces such as, for example, a femoral head and an acetabulum, one or both femoral condyle(s) and a tibial plateau, a trochlea and a patella, a glenoid and a humeral head, a talar dome and a tibial plafond, a distal humerus and a radial head and/or an ulna, or a radius and a scaphoid, certain embodiments allow for patient individualized, bone-preserving implant designs that can assist with proper ligament balancing and that can help avoid "overstuffing" of the joint, while achieving optimal bone preservation on one or more articular surfaces in each patient.

The resection cuts also can be designed to meet or exceed a certain minimum material thickness, for example, the minimum amount of thickness required to ensure biomechanical stability and durability of the implant. In certain embodiments, the limiting minimum implant thickness can be defined at the intersection of two adjoining bone cuts on the inner, bone-facing surface of an implant component. In certain embodiments of a femoral implant component, the minimum implant thickness can be less than 10 mm, less than 9 mm, less than 8 mm, less than 7 mm, and/or less than 6 mm. Optimized resection cuts for articular surfaces in knee replacement

In a knee, different resection cuts can be planned for a medial and lateral femoral condyle. In certain embodiments, a single bone cut can be optimized in a patient to maximize bone preservation in that select area, for example, a posterior condyle. Alternatively, multiple or all resection cuts can be optimized. Since a patient's medial and lateral femoral condyles typically have different geometries, including, for example, width, length and radii of curvature in multiple planes, for example, the coronal and the sagittal plane, then one or more resection cuts can be optimized in the femur individually for each condyle, resulting in resection cuts placed at a different depths, angles, and/or orientations in one condyle relative to the other condyle. For example, a horizontal cut in a medial condyle may be anatomically placed more inferior relative to the limb than a horizontal cut in a lateral condyle. The distance of the horizontal cut from the subchondral bone may be the same in each condyle or it can be different in each condyle. Chamfer cuts in the medial and lateral condyle may be placed in different planes rather than the same plane in order to optimize bone preservation. Moreover, chamfer cuts in the medial and lateral condyle may be placed at a different angle in order to maximize bone preservation. Posterior cuts may be placed in a different plane, parallel or non-parallel, in a medial and a lateral femoral condyle in order to maximize bone preservation. A medial condyle may include more bone cut facets than a lateral condyle in order to enhance bone preservation or vice versa.

In certain embodiments, a measure of bone preservation can include total volume of bone resected, volume of bone resected from one or more resection cuts, volume of bone resected to fit one or more implant component bone cuts, average thickness of bone resected, average thickness of bone resected from one or more resection cuts, average thickness of bone resected to fit one or more implant component bone cuts, maximum thickness of bone resected, maximum thickness of bone resected from one or more resection cuts, maximum thickness of bone resected to fit one or more implant component bone cuts.

Certain embodiments of femoral implant components described herein include more than five bone cuts, for example, six, seven, eight, nine or more bone cuts on the inner, bone-facing surface of the implant component. These bone cuts can be standard, in whole or in part, or patient-adapted, in whole or in part. Alternatively, certain embodiments include five bone cuts that are patient-adapted based on one or more images of the patient's knee. A femoral implant component with greater than five bone cuts of and/or with patient-adapted bone cuts can allow for enhanced bone preservation over a traditional femoral implant with five standard bone cuts and therefore can perform as a pre-primary implant.

In addition to optimizing bone preservation, another factor in determining the depth, number, and/or orientation of resection cuts and/or implant component bone cuts is desired implant thickness. A minimum implant thickness can be included as part of the resection cut and/or bone cut design to ensure a threshold strength for the implant in the face of the stresses and forces associated with joint motion, such as standing, walking, and running. Before, during, and/or after establishing a minimum implant component thickness, the optimum depth of the resection cuts and the optimum number and orientation of the resection cuts and bone cuts, for example, for maximum bone preservation, can designed.

In certain embodiments, an implant component design or selection can depend, at least in part, on a threshold minimum implant component thickness. In turn, the threshold minimum implant component thickness can depend, at least in part, on patient-specific data, such as condylar width, femoral transepicondylar axis length, and/or the patient's specific weight. In this way, the threshold implant thickness, and/or any implant component feature, can be adapted to a particular patient based on a combination of patient-specific geometric data and on patient-specific anthropometric data. This approach can apply to any implant component feature for any joint, for example, the knee, the hip, or the shoulder.

A weighting optionally can be applied to each bone with regard to the degree of bone preservation achieved. For example, if the maximum of bone preservation is desired on a tibia or a sub-segment of a tibia, femoral bone cuts can be adapted and moved accordingly to ensure proper implant alignment and ligament balancing. Conversely, if maximum bone preservation is desired on a femoral condyle, a tibial bone cut can be adjusted accordingly. If maximum bone preservation is desired on a patella, a resection cut on the opposing trochlea can be adjusted accordingly to ensure maximal patellar bone preservation without inducing any extension deficits. If maximum bone preservation is desired on a trochlea, a resection cut on the opposing patella can be adjusted accordingly to ensure maximal patellar bone preservation without inducing any extension deficits. Any combination is possible and different weightings can be applied. The weightings can be applied using mathematical models or, for example, data derived from patient reference databases.

### Ligament preservation

Implant design and modeling also can be used to achieve ligament sparing, for example, with regard to the PCL and/or the ACL. An imaging test can be utilized to identify, for example, the origin and/or the insertion of the PCL and the ACL on the femur and tibia. The origin and the insertion can be identified by visualizing, for example, the ligaments directly, as is possible with MRI or spiral CT arthrography, or by visualizing bony landmarks known to be the origin or insertion of the ligament such as the medial and lateral tibial spines.

An implant system can then be selected or designed based on the image data so that, for example, the femoral component preserves the ACL and/or PCL origin, and the tibial component preserves the ACL and/or PCL attachment. The implant can be selected or designed so that bone cuts adjacent to the ACL or PCL attachment or origin do not weaken the bone to induce a potential fracture.

For ACL preservation, the implant can have two unicompartmental tibial components that can be selected or designed and placed using the image data. Alternatively, the implant can have an anterior bridge component. The width of the anterior bridge in AP dimension, its thickness in the superoinferior dimension or its length in mediolateral dimension can be selected or designed using the imaging data and, specifically, the known insertion of the ACL and/or PCL.

### Establishing normal or near-normal joint kinematics

In certain embodiments, bone cuts and implant shape including at least one of a bone-facing or a joint-facing surface of the implant can be designed or selected to achieve normal joint kinematics.

In certain embodiments, a computer program simulating biomotion of one or more joints, such as, for example, a knee joint, or a knee and ankle joint, or a hip, knee and/or ankle joint can be utilized. In certain embodiments, patient-specific imaging data can be fed into this computer program. For example, a series of two-dimensional images of a patient's knee joint or a three-dimensional representation of a patient's knee joint can be entered into the program. Additionally, two-dimensional images or a three-dimensional representation of the patient's ankle joint and/or hip joint may be added.

Optionally, other data including anthropometric data may be added for each patient. These data can include but are not limited to the patient's age, gender, weight, height, size, body mass index, and race. Desired limb alignment and/or deformity correction can be added into the model. The position of bone cuts on one or more articular surfaces as well as the intended location of implant bearing surfaces on one or more articular surfaces can be entered into the model.

A patient-specific biomotion model can be derived that includes combinations of parameters listed above. The biomotion model can simulate various activities of daily life including normal gait, stair climbing, descending stairs, running, kneeling, squatting, sitting and any other physical activity. The biomotion model can start out with standardized activities, typically derived from reference databases. These reference databases can be, for example, generated using biomotion measurements using force plates and motion trackers using radio frequency or optical markers and video equipment.

The biomotion model can then be individualized with use of patient-specific information including at least one of, but not limited to the patient's age, gender, weight, height, body mass index, and race, the desired limb alignment or deformity correction, and the patient's imaging data, for example, a series of two-dimensional images or a three-dimensional representation of the joint for which surgery is contemplated.

An implant shape including associated bone cuts generated in the preceding optimizations, for example, limb alignment, deformity correction, bone preservation on one or more articular surfaces, can be introduced into the model. **Table 2** includes an exemplary list of parameters that can be measured in a patient-specific biomotion model.

**Table 2: Parameters measured in a patient-specific biomotion model for various implants**

| **Joint implant** | **Measured Parameter** |
|---|---|
| **knee** | Medial femoral rollback during flexion |
| **knee** | Lateral femoral rollback during flexion |
| **knee** | Patellar position, medial, lateral, superior, inferior for different flexion and extension angles |
| **knee** | Internal and external rotation of one or more femoral condyles |
| **knee** | Internal and external rotation of the tibia |
| **knee** | Flexion and extension angles of one or more articular surfaces |
| **knee** | Anterior slide and posterior slide of at least one of the medial and lateral femoral condyles during flexion or extension |
| **knee** | Medial and lateral laxity throughout the range of motion |
| **knee** | Contact pressure or forces on at least one or more articular surfaces, e.g. a femoral condyle and a tibial plateau, a trochlea and a patella |
| **knee** | Contact area on at least one or more articular surfaces, e.g. a femoral condyle and a tibial plateau, a trochlea and a patella |
| **knee** | Forces between the bone-facing surface of the implant, an optional cement interface and the adjacent bone or bone marrow, measured at least one or multiple bone cut or bone-facing surface of the implant on at least one or multiple articular surfaces or implant components. |
| **knee** | Ligament location, e.g. ACL, PCL, MCL, LCL, retinacula, joint capsule, estimated or derived, for example using an imaging test. |
| **knee** | Ligament tension, strain, shear force, estimated failure forces, loads for example for different angles of flexion, extension, rotation, abduction, adduction, with the different positions or movements optionally simulated in a virtual environment. |
| **knee** | Potential implant impingement on other articular structures, e.g. in high flexion, high extension, internal or external rotation, abduction or adduction or any combinations thereof or other angles / positions / movements. |
| **Hip, shoulder or other joint** | Internal and external rotation of one or more articular surfaces |
| **Hip, shoulder or other joint** | Flexion and extension angles of one or more articular surfaces |
| **Hip, shoulder or other joint** | Anterior slide and posterior slide of at least one or more articular surfaces during flexion or extension, abduction or adduction, elevation, internal or external rotation |
| **Hip, shoulder or other joint** | Joint laxity throughout the range of motion |
| **Hip, shoulder or other joint** | Contact pressure or forces on at least one or more articular surfaces, e.g. an acetabulum and a femoral head, a glenoid and a humeral head |
| **Hip, shoulder or other joint** | Forces between the bone-facing surface of the implant, an optional cement interface and the adjacent bone or bone marrow, measured at least one or multiple bone cut or bone-facing surface of the implant on at least one or multiple articular surfaces or implant components. |
| **Hip, shoulder or other joint** | Ligament location, e.g. transverse ligament, glenohumeral ligaments, retinacula, joint capsule, estimated or derived, for example using an imaging test. |
| **Hip, shoulder or other joint** | Ligament tension, strain, shear force, estimated failure forces, loads for example for different angles of flexion, extension, rotation, abduction, adduction, with the different positions or movements optionally simulated in a virtual environment. |
| **Hip, shoulder or other joint** | Potential implant impingement on other articular structures, e.g. in high flexion, high extension, internal or external rotation, abduction or adduction or elevation or any combinations thereof or other angles / positions / movements. |

- The above list is not meant to be exhaustive, but only exemplary. Any other biomechanical parameter known in the art can be included in the analysis.
- The resultant biomotion data can be used to further optimize the implant design with the objective to establish normal or near normal kinematics. The implant optimizations can include one or multiple implant components. Implant optimizations based on patient-specific data including image based biomotion data include, but are not limited to:
- Changes to external, joint-facing implant shape in coronal plane
- Changes to external, joint-facing implant shape in sagittal plane
- Changes to external, joint-facing implant shape in axial plane
- Changes to external, joint-facing implant shape in multiple planes or three dimensions
- Changes to internal, bone-facing implant shape in coronal plane
- Changes to internal, bone-facing implant shape in sagittal plane
- Changes to internal, bone-facing implant shape in axial plane
- Changes to internal, bone-facing implant shape in multiple planes or three dimensions
- Changes to one or more bone cuts, for example with regard to depth of cut, orientation of cut

Any single one or combinations of the above or all of the above on at least one articular surface or implant component or multiple articular surfaces or implant components.

When changes are made on multiple articular surfaces or implant components, these can be made in reference to or linked to each other. For example, in the knee, a change made to a femoral bone cut based on patient-specific biomotion data can be referenced to or linked with a concomitant change to a bone cut on an opposing tibial surface, for example, if less femoral bone is resected, the computer program may elect to resect more tibial bone.

Similarly, if a femoral implant shape is changed, for example on an external surface, this can be accompanied by a change in the tibial component shape. This is, for example, particularly applicable when at least portions of the tibial bearing surface negatively-match the femoral joint-facing surface.

Similarly, if the footprint of a femoral implant is broadened, this can be accompanied by a widening of the bearing surface of a tibial component. Similarly, if a tibial implant shape is changed, for example on an external surface, this can be accompanied by a change in the femoral component shape. This is, for example, particularly applicable when at least portions of the femoral bearing surface negatively-match the tibial joint-facing surface.

Similarly, if a patellar component radius is widened, this can be accompanied by a widening of an opposing trochlear bearing surface radius, or vice-versa.

Any combination is possible as it pertains to the shape, orientation, and size of implant components on two or more opposing surfaces.

By optimizing implant shape in this manner, it is possible to establish normal or near normal kinematics. Moreover, it is possible to avoid implant related complications, including but not limited to anterior notching, notch impingement, posterior femoral component impingement in high flexion, and other complications associated with existing implant designs.

Biomotion models for a particular patient can be supplemented with patient-specific finite element modeling or other biomechanical models known in the art. Resultant forces in the knee joint can be calculated for each component for each specific patient. The implant can be engineered to the patient's load and force demands. For instance, a 1251b. patient may not need a tibial plateau as thick as a patient with 280 1bs. Similarly, the polyethylene can be adjusted in shape, thickness and material properties for each patient. For example, a 3 mm polyethylene insert can be used in a light patient with low force and a heavier or more active patient may need an 8mm polymer insert or similar device.

### Selecting and/or designing an implant component and, optionally, related surgical steps and guide tools

In certain embodiments, the assessment process includes selecting and/or designing one or more features and/or feature measurements of an implant component and, optionally, of a corresponding resection cut strategy and/or guide tool that is adapted (e.g., patient-adapted based on one or more of a particular patient's biological features and/or feature measurements) to achieve or address, at least in part, one or more of the following parameters for the particular patient: (1) correction of a joint deformity; (2) correction of a limb alignment deformity; (3) preservation of bone, cartilage, and/or ligaments at the joint; (5) preservation, restoration, or enhancement of joint kinematics, including, for example, ligament function and implant impingement; and (7) preservation, restoration, or enhancement of other target features.

Preserving, restoring, or enhancing bone, cartilage, and/or ligaments can include, for example, identifying diseased tissue from one or more images of the patient's joint, identifying a minimum implant thickness for the patient (based on, for example, femur and/or condyle size and patient weight); virtually assessing combinations of resection cuts and implant component features, such as variable implant thickness, bone cut numbers, bone cut angles, and/or bone cut orientations; identifying a combination of resection cuts and/or implant component features that, for example, remove diseased tissue and also provide maximum bone preservation (i.e., minimum amount of resected bone) and at least the minimum implant thickness for the particular patient; and selecting and/or designing one or more surgical steps (e.g., one or more resection cuts), one or more guide tools, and/or one or more implant components to provide the resection cuts and/or implant component features that provide removal of the diseased tissue, maximum bone preservation, and at least the minimum implant thickness for the particular patient.

The assessment process can be iterative in nature. For example, one or more first parameters can be assessed and the related implant component and/or resection cut features and feature measurements tentatively or conditionally can be determined. Next, one or more second parameters can be assessed and, optionally, one or more features and/or feature measurements determined. Then, the tentative or conditional features and/or feature measurements for the first assessed parameter(s) optionally can be altered based on the assessment and optional determinations for the second assessed parameters. The assessment process can be fully automated or it can be partially automated allowing for user interaction. User interaction can be particularly useful for quality assurance purposes.

In the assessment, different weighting can be applied to any of the parameters or parameter thresholds, for example, based on the patient's age, the surgeon's preference or the patient's preference. Feedback mechanisms can be used to show the user or the software the effect that certain feature and/or feature measurement changes can have on desired changes to parameters values, e.g., relative to selected parameter thresholds. For example, a feedback mechanism can be used to determine the effect that changes in features intended to maximize bone preservation (e.g., implant component thickness(es), bone cut number, cut angles, cut orientations, and related resection cut number, angles, and orientations) have on other parameters such as limb alignment, deformity correction, and/or joint kinematic parameters, for example, relative to selected parameter thresholds.

### Setting and weighing parameters

As described herein, certain embodiments can apply modeling, for example, virtual modeling and/or mathematical modeling, to identify optimum implant component features and measurements, and optionally resection features and measurements, to achieve or advance one or more parameter targets or thresholds. For example, a model of patient's joint or limb can be used to identify, select, and/or design one or more optimum features and/or feature measurements relative to selected parameters for an implant component and, optionally, for corresponding resection cuts and/or guide tools. In certain embodiments, a physician, clinician, or other user can select one or more parameters, parameter thresholds or targets, and/or relative weightings for the parameters included in the model. Alternatively or in addition, clinical data, for example obtained from clinical trials, or intraoperative data, can be included in selecting parameter targets or thresholds, and/or in determining optimum features and/or feature measurements for an implant component, resection cut, and/or guide tool.

### Generating bone cuts and resected surfaces

In certain embodiments, the implant component includes one or more bone cuts on its bone-facing surface. Features of these bone cuts, and optionally features of corresponding resection cuts, can be optimized by a computer system based on patient-specific data. For example, the bone cut number and one or more bone cut planes, angles, or depths, as well as the corresponding resection cut number and one or more resection cut planes, angles, or depths, can be optimized, for example, to preserve the maximum amount of bone for each individual patient based on a series of two-dimensional images or a three-dimensional representation of the articular anatomy and geometry and/or on a target limb alignment and/or deformity correction. Optionally, one or more of the bone cut features and/or resection cut features can be adjusted by the operator.

### Libraries

As described herein, implants of various sizes, shapes, curvatures and thicknesses with various types and locations and orientations and number of bone cuts can be selected and/or designed and manufactured. The implant designs and/or implant components can be selected from, catalogued in, and/or stored in a library. The library can be a virtual library of implants, or components, or component features that can be combined and/or altered to create a final implant. The library can include a catalogue of physical implant components. In certain embodiments, physical implant components can be identified and selected using the library. The library can include previously-generated implant components having one or more patient-adapted features, and/or components with standard or blank features that can be altered to be patient-adapted. Accordingly, implants and/or implant features can be selected from the library.

Alternatively or in addition, one or more features of an implant component and/or implant procedure can be preoperatively derived from patient-specific data to provide a patient-engineered feature, for example, to optimize one or more parameters, such as one or more of the parameters described above. For example, in certain embodiments, a bone preserving femoral implant component can include an inner, bone-facing surface (i.e., superior surface) having one or bone cuts that, at least in part, are patient-derived, optionally together with matching patient-derived resection cuts, to minimize the amount of resected bone (and maximize the amount of retained bone), for example, on the patient's femur. This patient-engineered implant component feature can be preoperatively selected and/or designed based on the patient's joint dimensions as seen, for example, on a series of two-dimensional images or a three-dimensional representation generated, for example, from a CT scan or MRI scan.

### Patellar-engaging surface of femoral implant component

Patellar revision can be very challenging and bone preservation is preferred in the patella. In certain embodiments, two or more patellar resection facets and two or more patellar implant component bone cuts are employed to preserve patellar bone stock. One or both of the two or more patellar facets can be substantially tangent or parallel to the medial and/or lateral uncut patellar surfaces. Optionally, particularly with more than two patellar resection facets, facets can be substantially tangent or parallel to uncut patellar superior and/or inferior surfaces.

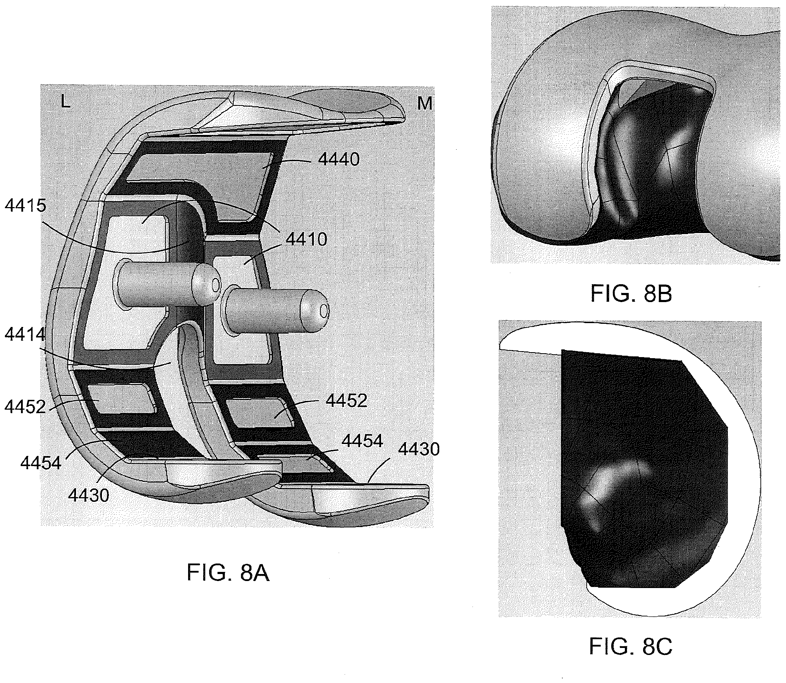

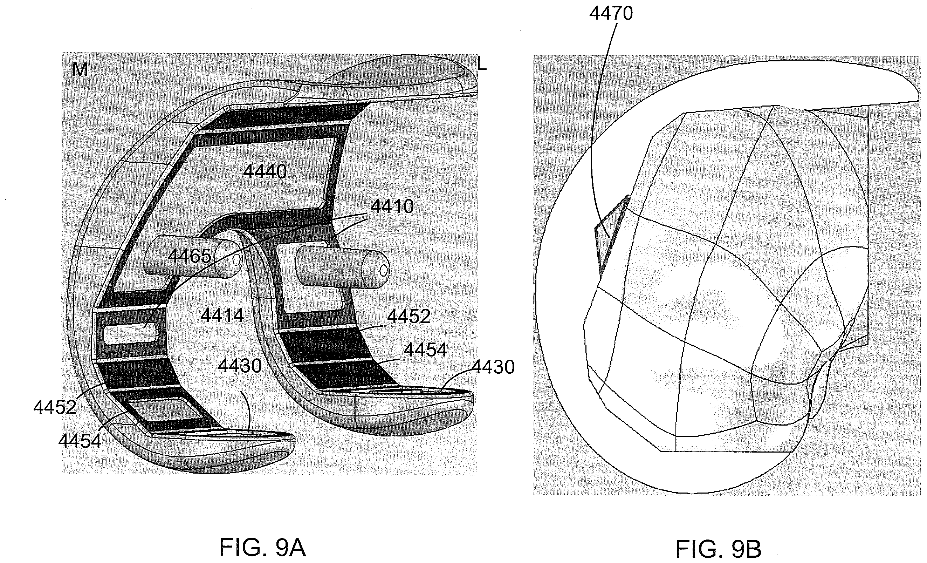

As shown in **FIGS. 8A-8C** and **8A-8B,** in certain embodiments an implant component bone cut can include one or more non-parallel and non-coplanar facets. As shown, the implant component in **FIG. 8A** includes six bone cuts while the implant component in **FIG. 8A** includes an extra posterior chamfer bone cut for a total of seven bone cuts. Non-coplanar facets can include facets that lie in parallel but different planes and/or facets that lie in non-parallel planes. As exemplified by the implant component shown in **FIGS. 8A-8B** and by the implant component shown in **FIGS. 8A-8B****,** the medial and lateral facets of a bone cut can be non-parallel and non-coplanar for one or more bone cuts, for example, for one or more of the distal bone cut **4410,** the posterior bone cut **4430,** the first posterior chamfer bone cut **4452,** and the second posterior chamfer bone cut **4454.** Alternatively or in addition, one or more corresponding facets of a bone cut can include different thicknesses. For example, the implant component shown in **FIG. 8A** includes a maximum distal medial facet thickness of 6.2 mm and a maximum distal lateral facet thickness of 6.3 mm. The independent and optionally patient-derived thicknesses on corresponding bone cut facets can apply to one or more thickness measurements, for example, one or more of maximum thicknesses, minimum thickness, and an average thickness, for example, to match or optimize the particular patient's anatomy. Moreover, a single bone cut or bone cut facet can include a variable thickness (e.g., a variable thickness profile across its M-L and/or A-P dimensions. For example, a bone cut or bone cut facet can include a thickness profile in one or more dimensions that is patient-derived (e.g., to match or optimize the patient's anatomy). The implant component in **FIG. 9A** includes an anterior chamfer with an 11 mm thickness on the medial side (which includes the medial implant component peg or post **4465**) and a different thickness on the lateral side. As shown, the implant component in **FIG. 9A** was selected and/or designed to have a flex-fit (e.g., having bone cuts rotated posteriorly about the transepicondylar axis, which can enhance implant component coverage of the posterior portion of the femur and provide the patient with deeper knee flexion with the implant.

Alternatively or in addition, one or more corresponding facets of a bone cut can include different surface areas or volumes. For bone cuts having facets separated by the intercondylar space and asymmetric with respect to the A-P plane bisecting the implant component, the asymmetric facets appear dissimilar in shape and/or size (e.g., two-dimensional area). For example, the implant components shown in **FIGS. 8A** and **9A** include one or more corresponding facets (e.g., distal medial and lateral facets, posterior medial and lateral facets, and/or posterior chamfer medial and lateral facets) having different medial facet and lateral facet bone-facing surface areas, joint-facing surface areas, and/or volumes in between the two surfaces. In particular, as shown in **FIGS. 8A** and in **9A,** the medial and lateral facets of the distal bone cut **4410** are asymmetric and appear dissimilar in both shape

(e.g., surface area perimeter shape) and size (e.g., volume under the surface area). The independent facet surface areas and/or volumes optionally can be patient-derived (e.g., to match or optimize the patient's anatomy).

As shown in **FIG. 8A****,** non-coplanar facets can be separated by an intercondylar space **4414** and/or by a step cut **4415.** For example, as shown in the figure, the distal bone cut **4410** includes non-coplanar medial and lateral facets that are separated, in part, by the intercondylar space **4414** and, in part, by a step cut **4415.**

In certain embodiments, one or more resection cuts can be selected and/or designed to so that one or more resected cut or facet surfaces substantially matches one or more corresponding bone cuts or bone cut facets. For example, **FIG. 8C** shows six resection cut facets that substantially match the corresponding implant component bone cut facets shown in **FIG. 8A****.** **FIG. 9B** shows seven resection cut facets that substantially match the corresponding implant component bone cut facets of the medial side of the implant component shown in **FIG. 8A****.** The portion 4470 represents additional bone conserved on the lateral side of the of the femur corresponding to the bone-cut intersection between the lateral distal bone cut facet **4410** and the adjacent anterior chamfer bone cut **4440.**

In addition or alternatively, in certain embodiments one or more of the implant bone cuts can be asymmetric, for example, asymmetric with respect to a sagittal or A-P plane, or to a coronal or M-L plane, bisecting the implant component. For example, as shown in **FIGS. 8A** and **9A****,** the anterior chamfer bone cut **4440** is asymmetric with respect to an A-P plane bisecting the implant component. In addition, in both figures the lateral distal bone cut facet **4410** is is asymmetric with respect to an A-P plane bisecting the implant component.

The bone cut designs described above, for example, an implant component bone-facing surface having various numbers of bone cuts, flexed bone cuts, non-coplanar bone cut facets, step cuts used to separate facets, and asymmetric bone cuts and bone cut facets, can be employed on a bone-facing surface of a femoral implant component to save substantial portions of bone. **Table 3** shows the different amounts of bone to be resected from a patient for fitting an implant component with bone cuts shown in **FIG. 8A****,** an implant component with bone cuts shown in **FIG. 8A****,** and a traditional implant component with traditional bone cuts. As compared to the traditional implant component, the implant component shown in **FIG. 8A** saves 44% of resected patient bone and the implant component shown in **FIG. 9A** saves 38% of resected patient bone. However, the implant component in **FIG. 9A** is flexed, which can provide enhanced deep knee flexion for the patient. In certain embodiments, the surgeon or operator can select or apply a weighting to these two parameters (bone preservation and kinematics) and optionally other parameters to identify an optimum patient-adapted implant component and, optionally, a corresponding resection cut strategy, that meets the desired parameters and/or parameter weightings for the particular patient.

**Table 3: Bone preservation comparison using different bone cut designs**

| **Bone Cut** | **FIG. 9D Implant (Stepped)** | **FIG. 9E Implant (Flexed no step)** | **Traditional Implant** |
|---|---|---|---|
| **Distal Medial** | 3626 | 2675 | 5237 |
| **Distal Lateral** | 2593 | 3077 | 2042 |
| **Medial Posterior Chamfer 1** | 942 | 694 | 3232 |
| **Lateral Posterior Chamfer 1** | 986 | 715 | 648 |
| **Medial Posterior Chamfer 2** | 816 | 921 | -- |
| **Lateral Posterior Chamfer 2** | 612 | 582 | -- |
| **Medial Posterior Cut** | 945 | 1150 | 2816 |
| **Lateral Posterior Cut** | 770 | 966 | 649 |
| **Anterior Chamfer 1** | 1667 | 6081 | 9872 |
| **Anterior Chamfer 2** | -- | 1845 | -- |
| **Anterior Cut** | 3599 | 2717 | 4937 |
| **Total bone resection (mm³)*** | 16556 | 18346 | 29433 |
| **Reduction in bone resected as compared to traditional implant** | 44% | 38% | -- |

### Anterior bone cut

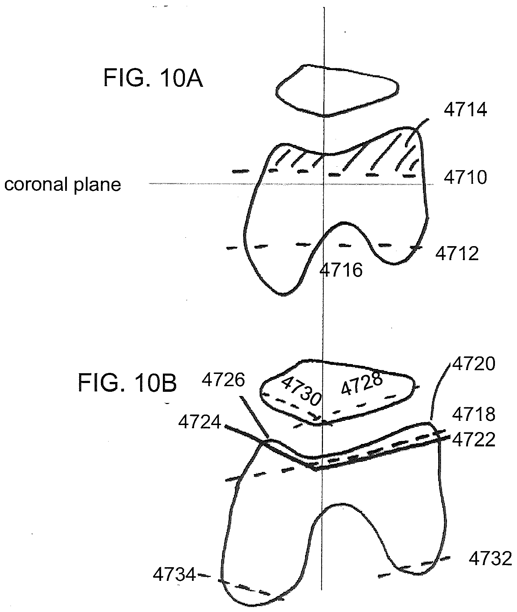

In traditional femoral implant components, the anterior or trochlear bone cut is located substantially in the coronal plane and is parallel to the posterior condylar bone cut, as indicated by the dashed anterior resection cut **4710** and dashed posterior resection cut **4712** shown in **FIG. 10A****.** This can result in a substantial amount of bone lost from those portions of the patient's femur **4714, 4716.** However, in certain embodiments described herein, the implant's anterior or trochlear bone cut is substantially non-parallel to the coronal plane, as shown by the dashed and straight line **4718** in **FIG. 10B****.** For example, the implant's anterior or trochlear bone cut can substantially match the patient's resected trochlear surface, which can be selected and/or designed to be parallel to a tangent through the corresponding peak **4720** and an uncut trochlear surface portion of the patient's trochlea, as shown in **FIG. 10B****.** By placing the implant bone cut **4718** and the resected surface at an angle relative to the patient's coronal plane, for example, parallel to a tangent of one or both medial and lateral trochlear peak and/or the adjacent trochlear surface, a substantial amount of bone can be preserved.

In certain embodiments, the implant component can include a trochlear bone cut with two or more non-coplanar facets, as shown by the intersecting solid lines **4722, 4724** in **FIG. 10B****.** For example, one of the two or more facets (and the patient's corresponding resected surface) can be substantially parallel to the patient's lateral uncut trochlear peak **4720** and/or the adjacent uncut trochlear surface. A second facet (and the patient's corresponding resected surface) can be substantially parallel to the patient's medial uncut trochlear peak **4726** and/or the adjacent uncut trochlear surface. This can further enhance the degree of bone preservation.

In certain embodiments, two or more trochlear bone cut facets can be substantially tangent to the lateral and medial patellar surfaces **4728, 4730** of the patient's uncut bone. In addition or alternatively, two or more trochlear bone cuts can be substantially tangent or parallel to the superior and inferior patellar facets, in particular, when more than two implant trochlear bone cut facets are used. In certain embodiments, one or more trochlear bone cut facets can be curvilinear.

### Posterior bone cut

In a traditional femoral implant component, the posterior bone cut includes portions on the medial and lateral condyles that are in the same plane and parallel to each other, and substantially parallel to the anterior cut. However, in certain embodiments described herein, the implant component can include posterior condylar bone cut facets on the medial and lateral condyles, respectively, that are non-coplanar **4732, 4734.** Alternatively, or additionally, the implant component can include one or more posterior condylar facets that are substantially non-parallel with one or more facts of the anterior bone cut.

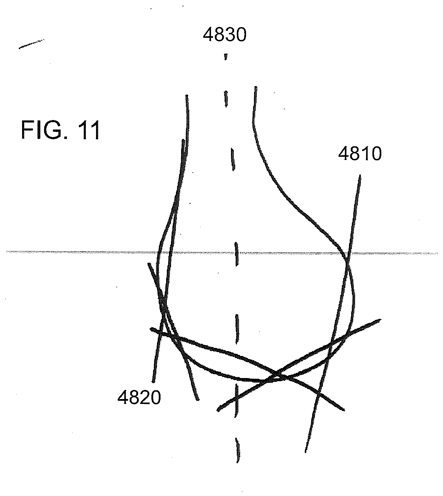

In certain preferred embodiments, the posterior condylar bone cut includes a facet on the medial condyle that is substantially perpendicular to the long axis of the medial condyle. Optionally, the facet on the lateral condyle can be perpendicular to the long axis of the lateral condyle. As depicted in **FIG. 11****,** in certain embodiments, the anterior bone cut and corresponding resection cut **4810** and posterior bone cut **4820** can be substantially non-parallel to the coronal plane **4830** in the superoinferior orientation.

### Distal bone cut

In certain embodiments, the distal bone cut of a femoral implant component includes medial and lateral condylar facets that are in the same plane as each other and/or are substantially parallel to each other. The facets can be separated by the intercondylar space and/or by a step cut. In certain embodiments, the implant component can include a distal bone cut having medial and lateral condylar facets that are non-coplanar and/or non-parallel.

In certain embodiments, the distal bone cut or bone cut facets is/are asymmetric with respect to an A-P plane bisecting the implant component. Moreover, the distal bone cut and/or one or more distal bone cut facets can be curvilinear.

### Chamfer bone cuts

Traditional femoral implant components include one anterior chamfer bone cut and one posterior chamfer bone cut. However, in certain embodiments described herein, additional chamfer bone cuts can be included. By increasing the number of chamfer bone cuts on the implant and placing the cuts in close proximity to the tangent of the articular surface, additional bone can be preserved. One or more additional chamfer bone cuts can be substantially tangent to the articular surface. For example, in certain embodiments, the implant component can include one or more additional anterior chamfer cuts and/or one or more additional posterior chamfer cuts.

In certain embodiments, the implant component can include a posterior chamfer bone cut that includes medial and lateral condylar facets that are non-coplanar and/or non-parallel. In certain embodiments, a posterior chamfer bone cut of the implant component can include facets that are asymmetric with respect to an A-P plane bisecting the implant component. Moreover, one or more posterior chamfer bone cuts and/or one or more posterior chamfer bone cut facets can be curvilinear.

In certain embodiments, the implant component can include an anterior chamfer bone cut that includes medial and lateral condylar facets that are non-coplanar and/or non-parallel. In certain embodiments, an anterior chamfer bone cut of the implant component can be asymmetric and/or can include facets that are asymmetric with respect to an anterior-posterior (A-P) plane bisecting the implant component. Moreover, one or more anterior chamfer bone cuts and/or bone cut facets can be curvilinear.

### Cut strategies

Computer software can be used that calculates the closest location possible for resected surfaces and resected cuts relative to the articular surface of the uncut bone, e.g., so that all intersects of adjoining resected surfaces are just within the bone, rather than outside the articular surface. The software can move the cuts progressively closer to the articular surface. When all intersects of the resected cuts reach the endosteal bone level, the subchondral bone level, and/or an established threshold implant thickness, the maximum exterior placement of the resected surfaces is achieved and, with that, the maximum amount of bone preservation.

### Examples

Examples not falling within the scopes of the claims are for illustrative purposes only.

### Example 1: Exemplary design process for certain patient-specific total knee implants

**Example 1** describes an exemplary process for designing a patient-adapted implant component. **Example 2** describes an exemplary patient-adapted knee implants components and methods for designing the same. Example 3 describes exemplary knee implants components having patient-adapted features and non-traditional features

This example describes an exemplary process for selecting and/or designing a patient-adapted total knee implant, for example, a knee implant having one or more patient-specific and/or patient-engineered based on patient-specific data. The steps described in this process can be performed in any order and can be performed more than once in a particular process. For example, one or more steps can be reiterated and refined a second, third, or more times, before, during, or after performing other steps or sets of steps in the process. While this process specifically describes steps for selecting and/or designing a patient-specific total knee implant, it can be adapted to design other embodiments, for example, patient-adapted bicompartmental knee implants, unicompartmental knee implants, and implants for shoulders and hips, vertebrae, and other joints.

### Methods

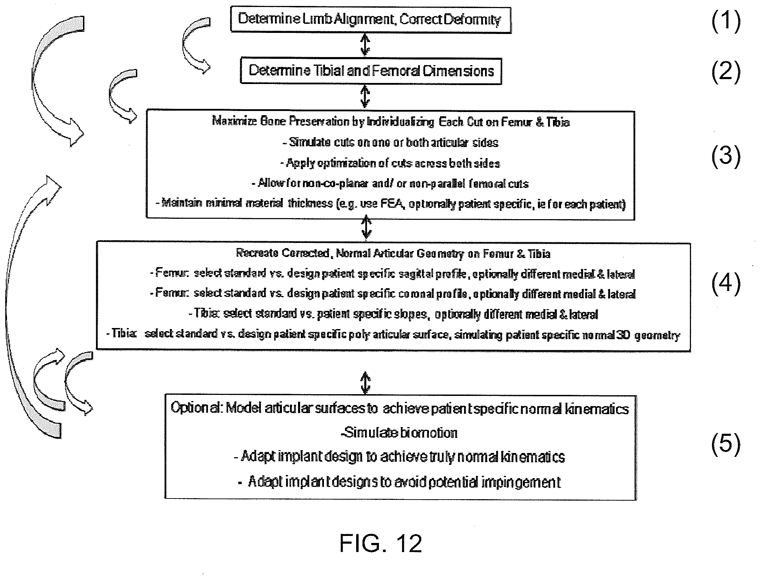

The exemplary process shown in **FIG. 12** includes four general steps and, optionally, can include a fifth general step. Each general step includes various specific steps. The general steps are identified as (1)-(5) in the figure. These steps can be performed virtually, for example, by using one or more computers that have or can receive patient-specific data and specifically configured software or instructions to perform such steps.

In general step (1), limb alignment and deformity corrections are determined, to the extent that either is needed for a specific patient's situation. In general step (2), the requisite tibial and femoral dimensions of the implant components are determined based on patient-specific data obtained, for example, from image data of the patient's knee.

In general step (3), bone preservation is maximized by virtually determining a resection cut strategy for the patient's femur and/or tibia that provides minimal bone loss optionally while also meeting other user-defined parameters such as, for example, maintaining a minimum implant thickness, using certain resection cuts to help correct the patient's misalignment, removing diseased or undesired portions of the patient's bone or anatomy, and/or other parameters. This general step can include one or more of the steps of (i) simulating resection cuts on one or both articular sides (e.g., on the femur and/or tibia), (ii) applying optimized cuts across one or both articular sides, (iii) allowing for non-co-planar and/or non-parallel femoral resection cuts (e.g., on medial and lateral corresponding portions of the femur) and, optionally, non-co-planar and/or non-parallel tibial resection cuts (e.g., on medial and lateral corresponding portions of the tibia), and (iv) maintaining and/or determining minimal material thickness. The minimal material thickness for the implant selection and/or design can be an established threshold, for example, as previously determined by a finite element analysis ("FEA") of the implant's standard characteristics and features. Alternatively, the minimal material thickness can be determined for the specific implant, for example, as determined by an FEA of the implant's standard and patient-specific characteristics and features. This step identifies for a surgeon the bone resection design to perform in the surgical theater and it also identifies the design of the bone-facing surface(s) of the implant components, which substantially negatively-match the patient's resected bone surfaces, at least in part.

In general step (4), a corrected, normal and/or optimized articular geometry on the femur and tibia is recreated virtually. For the femur, this general step can include, for example, the step of: (i) selecting a standard sagittal profile, or selecting and/or designing a patient-engineered or patient-specific sagittal profile; and (ii) selecting a standard coronal profile, or selecting and/or designing a patient-specific or patient-engineered coronal profile. Optionally, the sagittal and/or coronal profiles of one or more corresponding medial and lateral portions (e.g., medial and lateral condyles) can include different curvatures. For the tibia, this general step includes one or both of the steps of: (iii) selecting a standard anterior-posterior slope, and/or selecting and/or designing a patient-specific or patient-engineered anterior-posterior slope, either of which optionally can vary from medial to lateral sides; and (iv) selecting a standard poly-articular surface, or selecting and/or designing a patient-specific or patient-engineered poly-articular surface. The patient-specific poly-articular surface can be selected and/or designed, for example, to simulate the normal or optimized three-dimensional geometry of the patient's tibial articular surface. The patient-engineered poly-articular surface can be selected and/or designed, for example, to optimize kinematics with the bearing surfaces of the femoral implant component. This step can be used to define the bearing portion of the outer, joint-facing surfaces (i.e., articular surfaces) of the implant components.

In optional general step (5), a virtual implant model (for example, generated and displayed using a computer specifically configured with software and/or instructions to assess and display such models) is assessed and can be altered to achieve normal or optimized kinematics for the patient. For example, the outer joint-facing or articular surface(s) of one or more implant components can be assessed and adapted to improve kinematics for the patient. This general step can include one or more of the steps of: (i) virtually simulating biomotion of the model, (ii) adapting the implant design to achieve normal or optimized kinematics for the patient, and (iii) adapting the implant design to avoid potential impingement.

### Results and discussion

The exemplary process described above yields both a predetermined surgical resection design for altering articular surfaces of a patient's bones during surgery and a design for an implant that specifically fits the patient, for example, following the surgical bone resectioning. Specifically, the implant selection and/or design, which can include manufacturing or machining the implant to the selected and/or designed specifications using known techniques, includes one or more patient-engineered bone-facing surfaces that negatively-match the patient's resected bone surface. The implant also can include other features that are patient-adapted, such as minimal implant thickness, articular geometry, and kinematic design features. This process can be applied to various joint implants and to various types of joint implants. For example, this design process can be applied to a total knee, cruciate retaining, posterior stabilized, and/or ACL/PCL retaining knee implants, bicompartmental knee implants, unicompartmental knee implants, and other joint implants, for example, for the shoulder, hip, elbow, spine, or other joints.

The exemplary process described above, including the resulting patient-adapted implants and predetermined bone resectioning design, offers several advantages over traditional primary and revision implants and related processes. For example, it allows for one or more pre-primary implants such that a subsequent replacement or improvement can take the form of a primary implant. Specifically, because the process described herein can minimize the amount of bone that is resected, enough bone stock may remain such that a subsequent procedure may be performed with a traditional, primary, off-the-shelf implant. This offers a significant advantage for younger patients who may require in their lifetime more than a single revision for an implant. In fact, the exemplary process described above may allow for two or more pre-primary implants or procedures before so much bone stock is sacrificed that a traditional, primary implant is required.

The advantageous minimal bone resectioning and therefore minimal bone loss that is achieved with this process arises from the fact that the bone-facing surfaces of the implants are derived for each patient based on patient-specific data, such as, for example, data derived from images of the patient's joint, size or weight of the patient, size of the joint, and size, shape and/or severity of defects and/or disease in the joint. This patient-adapted approach allows for the bone-facing surface of the implant components to be optimized with respect to any number of parameters, including minimizing bone loss, using any number of resection cuts and corresponding implant component bone cuts and bone cut facets to conserve bone for the patient. With traditional implants, the implant's bone-facing surface includes standard bone cuts and the resection cuts to the patient's bone are made to fit those standard bone cuts.

Another advantage to this process is that the selection and/or design process can incorporate any number of target parameters such that any number of implant component features and resection cuts can be selected and/or designed to meet one or more parameters that are predetermined to have clinical value. For example, in addition to bone preservation, a selection and/or design process can include target parameters to restore a patient's native, normal kinematics, or to provide optimized kinematics. For example, the process for selecting and/or designing an implant and/or resection cuts can include target parameters such as reducing or eliminating the patient's mid-flexion instability, reducing or eliminating "tight" closure, improving or extending flexion, improving or restoring cosmetic appearance, and/or creating or improving normal or expected sensations in the patient's knee. The design for a tibial implant can provide an engineered surface that replicates the patient's normal anatomy yet also allows for low contact stress on the tibia.

For surgeons and medical professionals, this process also provides a simplified surgical technique. The selected and/or designed bone cuts and, optionally, other features that provide a patient-adapted fit for the implant components eliminates the complications that arise in the surgical setting with traditional, misfitting implants. Moreover, since the process and implant component features are predetermined prior to surgery, model images of the surgical steps can be provided to the surgeon as a guide.

As noted above, the design of an implant component can include manufacturing or machining the component in accordance with the implant design specifications. Manufacturing can include, for example, using a designed mold to form the implant component. Machining can include, for example, altering a selected blank form to conform to the implant design specifications. For example, using the steps described above, the femoral implant component can be manufactured from a designed mold and the tibial implant component, including each of a tibial tray and insert, can be customized from a selected starting tray and insert, for example, from blanks.

### Example 2: Patient-adapted femoral implant component with five bone cuts and corresponding resection cuts

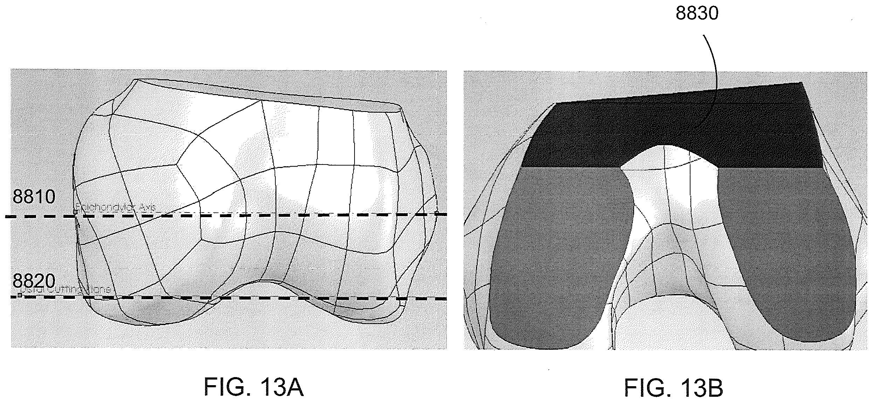

This example describes two exemplary methods for designing resection cuts to a patient's femur and related bone cuts on the bone-facing surface of a femoral implant component designed for the patient. In particular, in both methods, a model of a patient's distal femur is created based on data from patient-specific two- or three-dimensional images of the patient's femur. As shown in **FIG. 13A****,** the epicondylar axis **8810** is determined for the patient's femur. Then, the resection cut planes and cut angles (and corresponding implant component cut planes and cut angles) are assessed and selected using the epicondylar axis **8810** as a reference. Specifically, four of the five cut planes - the distal cut, posterior cut, posterior chamfer cut, and anterior chamfer cut - are designed to be parallel with the epicondylar axis **8810.** **FIG. 13A** shows the distal cut plane **8820** parallel to the epicondylar axis **8810.** However, for the particular patient, the anterior cut plane is designed to be oblique to the epicondylar axis **8810,** which can minimize the amount of bone resected on the lateral side of the cut. **FIG. 13B** shows an example of an anterior oblique cut plane **8830.**

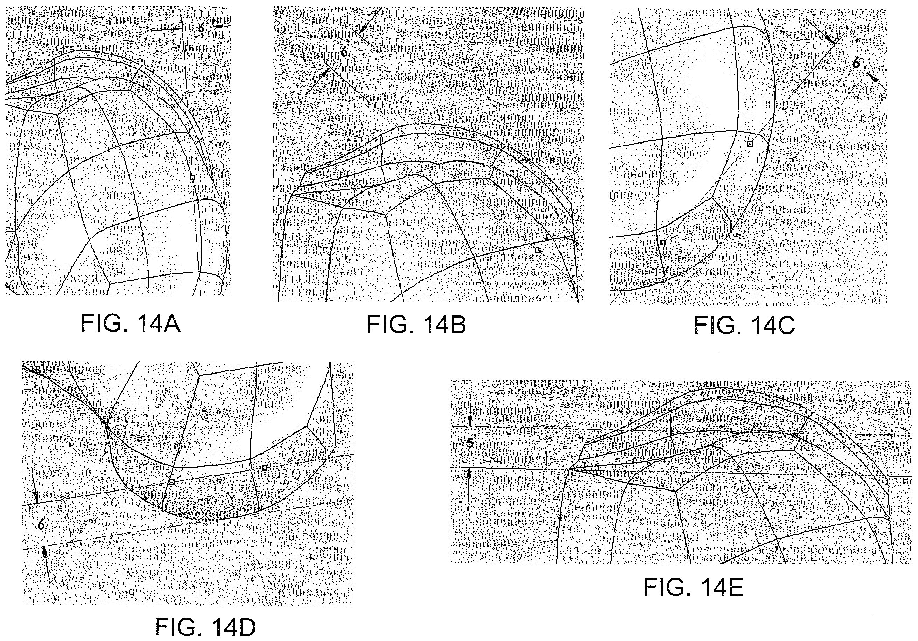

For each of the five cut planes, optimized cuts (i.e., resection cuts) tangent to the bone surface at the angle of each resection plane also is determined. The optimized cuts as shown in **FIGS. 14A** to **14E** included a maximum cut depth of 6 mm for the distal cut plane (FIG. **14A****),** the anterior chamfer cut plane (**FIG. 14B**), the posterior chamfer cut (**FIG. 14C**), and the posterior cut plane (**FIG. 14D**). The maximum cut depth is 5 mm for the anterior cut plane (**FIG. 14E**). Optimized cuts can be determined based on one or more parameters, including those described above. In this example, optimized cut were determined, at least in part, to minimize resected bone while providing greater than a threshold minimum implant thickness. Deeper resection cuts allow for a thicker implant, but require greater bone loss. Typically, the thinnest resection cut depth and, accordingly, the minimum implant thickness occurs at the intersections between cut planes. Accordingly, alternatively or in addition to altering cut plane depths, the number of cut planes, the cut plane angles and/or the cut plane orientations can be altered to provide deeper cut plane intersections and corresponding greater minimum implant thickness at the bone cut intersections while also minimizing the amount of bone resected from the patient.

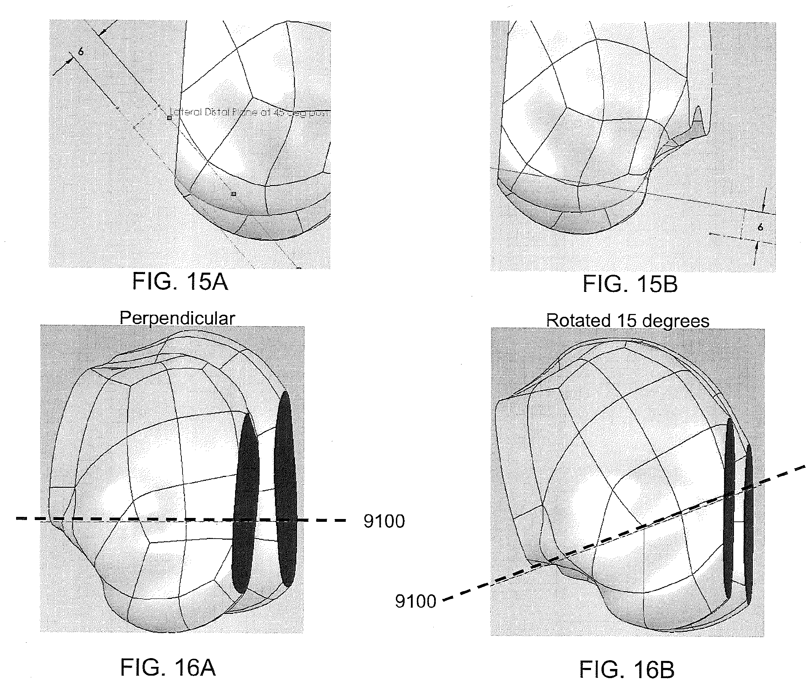

The optimized number of cut planes, depths of cut planes, angles of cut planes and/or orientations of cut planes can be determined independently for each of the medial and lateral femoral condyles. For example, **FIGS. 14A** to **14E** show optimized cut planes based on the medial condyle. However, **FIGS. 15A** and **15B** show cut planes for the lateral condyle posterior chamfer **(****FIG. 15A**) and lateral condyle posterior **(****FIG. 15B****)** cut planes that are independently optimized (i.e., relative to the medial condyle posterior chamfer and medial condyle posterior cut planes, respectively) based on patient-specific data for the lateral condyle. This type of independent optimization between condyles can result in a different number of cut plane facets, different angles of cut plane facets, and/or different depths of cut plane facets on corresponding portions of medial and lateral condyles.

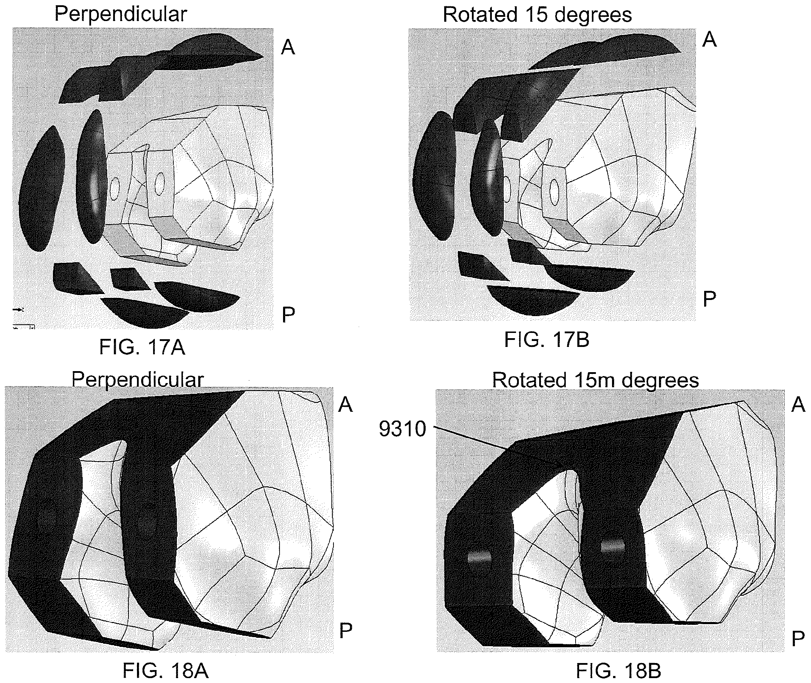

Two exemplary resection cut designs (and corresponding implant component bone cut design e.g., that includes substantially matching cut features of the resection cut design) is based on five cut planes are described in this example. In the first design, shown in **FIG. 16A****,** a distal cut plane is designed perpendicular to the sagittal femoral axis **9100.** In the second design, referred to as a "flexed" or "flex-fit" design" and shown in **FIG. 16B****,** the distal cut plane is rotated 15 degrees in flexion from the perpendicular to the sagittal femoral axis. The additional four cut planes are shifted accordingly for each design method, as shown in **FIGS. 17A** and **17B****.**

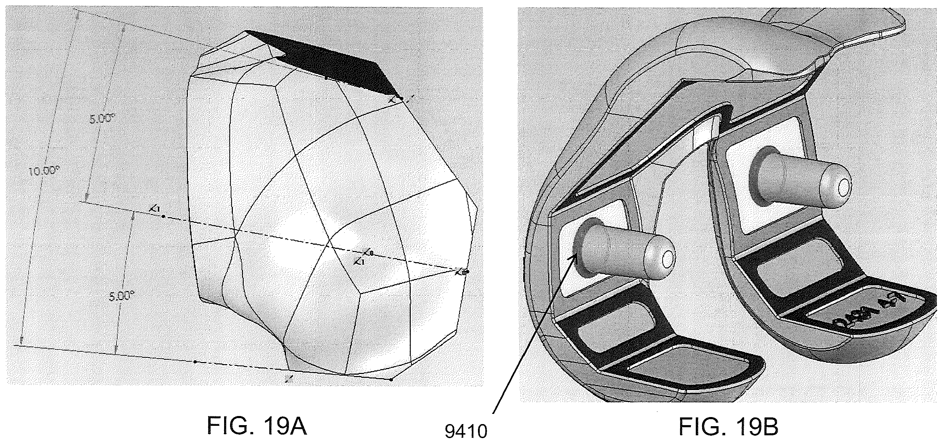

**FIGS. 18A** and **18B** show the completed cut femur models for each cut design. For each design, the maximum resection depth for each cut plane was 6 mm, except for the anterior cut plane, which was 5 mm. The "flex-fit" design can provide more posterior coverage in high flexion. However, it also may require more anterior bone resectioning to achieve sufficient coverage and may require particular attention during actual bone cutting to avoid any incomplete bone removal at the trochlear notch **9310.** In certain embodiments of a cut plane design, the anterior and posterior cut planes diverge from the component peg axis by five degrees each, as shown in **FIG. 19A****.** With a traditional femoral implant component, the posterior and anterior cut planes diverge 2 degrees and 7 degrees, respectively, from the peg axis. Moreover, in certain embodiments, the peg can be designed to have various dimensions. For example, the design in **FIG. 19B** includes a peg diameter of 7 mm tapering to about 6.5 mm, a length of 14 mm with a rounded tip, and a base with a 1 mm fillet **9410.**

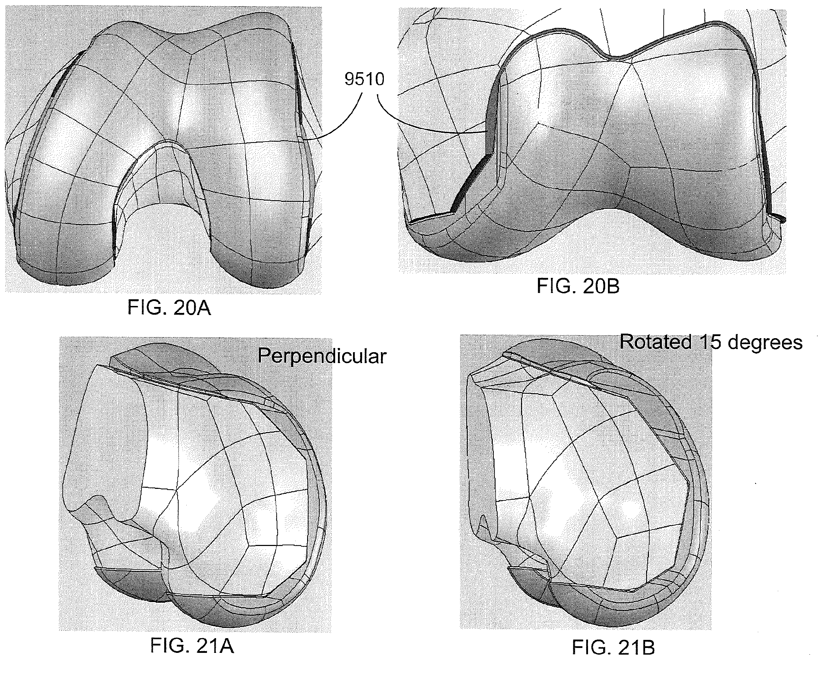

An exemplary bone facing surface of the femoral implant component design is shown in **FIGS. 20A** and **20B****.** In addition to optimized cut planes described above, these implant components also include a patient-adapted peripheral margin **9510** that is 0.5 mm from the edge of cut bone. The designs also can include engineered coronal curvatures on the condyles. Side views of the resulting femoral implant component designs for the first and second design methods are shown in **FIGS. 21A** and **21B****.** This sagittal view of the implant components shows the difference in anterior and posterior coverage for the two designs. As seen by a comparison of the two figures, the flexed cut design provides greater posterior coverage, which enhances deep knee flexion for the patient. Accordingly, as shown by this example, one or more features or measurements derived from patient-specific data are used to preoperatively select and/or design implant component features that target and achieve more than one parameter, in this case preservation of the patient's bone and preservation, restoration, or enhancement of the patient's joint kinematics.

As mentioned above, the optimization of resection cuts and implant component bone cuts can result in a cut design that has any number of cut planes or facets, depths of cut planes or facets, angles of cut planes or facets, and/or orientations of cut planes or facets. In addition to optimizing the cut planes to minimize bone loss and maximize implant thickness, various other parameters can be included in the cut plane optimization. In this example, the flexed cut design was used to help preserve, restore, or enhance the patient's joint kinematics. Additional parameters that may be included in the determination of optimized resection and bone cuts can include, but are not limited to, one or more of: (1) deformity correction and/or limb alignment (2) maximizing preservation of cartilage, or ligaments, (3) maximizing preservation and/or optimization of other features of the patient's anatomy, such as trochlea and trochlear shape, (4) further restoration and/or optimization of joint kinematics or biomechanics (5) restoration or optimization of joint-line location and/or joint gap width, and (6) preservation, restoration, or enhancement of other target features.

### Example 3: Design of a femoral component of a total knee replacement with a bone-facing surface that optimizes bone preservation

This example describes an exemplary design of femoral implant component. In particular, the femoral component includes seven bone cuts on its inner, bone-facing surface.

### Methods

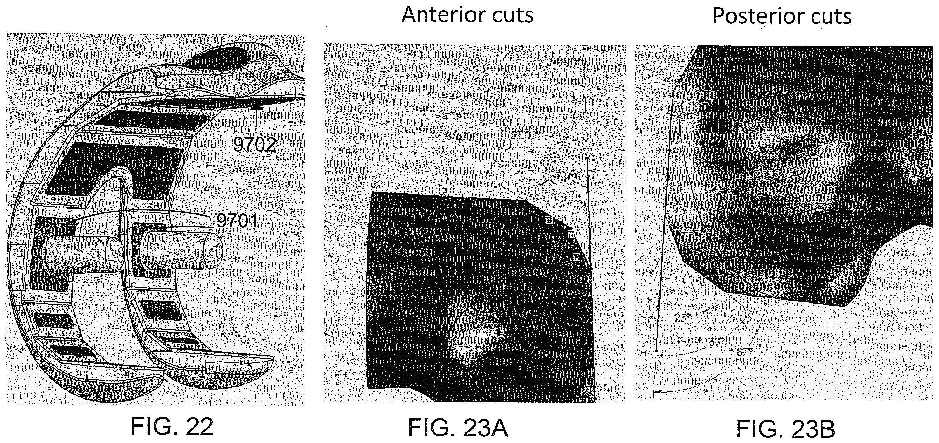

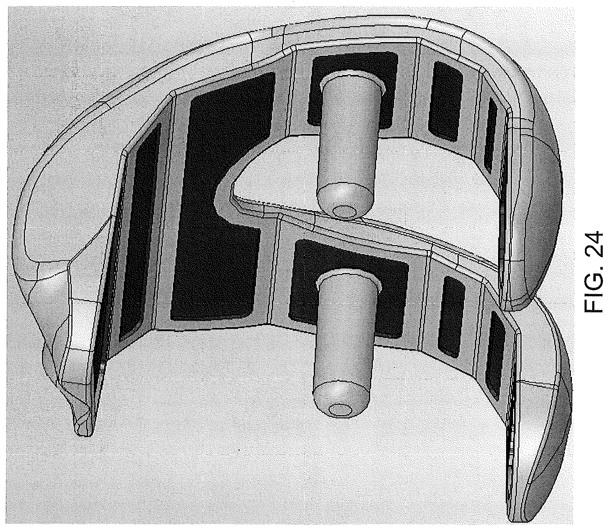

A femoral implant component (PCL-retaining) is designed with seven bone cuts for a femur-first implantation technique. The design of the implant component is depicted in **FIG. 22****.** The seven bone cuts on the inner, bone-facing surface of the implant component include a distal bone cut **9701** (including lateral and medial facets) that is perpendicular to the sagittal femoral axis, and an anterior cut **9702.** The corresponding resection cut planes are shown in **FIG. 23A** and in **FIG. 23B****.** Specifically, a first anterior chamfer cut plane is at 25 degrees, a second anterior chamfer cut plane is at 57 degrees, and an anterior cut plane is at 85 degrees relative to the distal femoral cut plane, as shown in **FIG. 23A**. Moreover, a first posterior chamfer cut plane is at 25 degrees, a second posterior chamfer cut plane is at 57 degrees, and a posterior chamfer cut plane is at 87 degrees relative to the distal femoral cut plane, as shown in **FIG. 23B****.** The femoral implant includes bone cuts that substantially negatively-match (e.g., in cut angle, area, and/or orientation) the resection cuts on these cut planes. The femoral implant component also can include on its bone-facing surface cement cutouts 9704 that are 0.5 mm deep and offset from the outer edge by 2 mm, and a peg protruding from the each of the lateral and medial distal bone cuts facets. The pegs are 7 mm in diameter, 17 mm long and are tapered by 0.5 degrees as they extend from the component. **FIG. 24** shows the cement pocket and peg features.

### Results and Discussion

In a traditional femoral implant component, the bone-facing surface consists of five standard bone cuts. However, the femoral component in this example includes seven bone cuts on the bone-facing surface. The additional bone cuts can allow for the corresponding resection cut planes be less deep from the bone surface to insure that the cut plane intersections have a depth below the bone surface that allows for a minimum implant thickness. Accordingly, less bone can be resected for a seven-bone-cut implant component than for a traditional five-bone-cut implant component to provide the same minimum implant component thickness, e.g., at the bone cut intersections. Moreover, the outer, joint-facing surface of the implant component described in this example includes a combination of patient-adapted features and standard features.

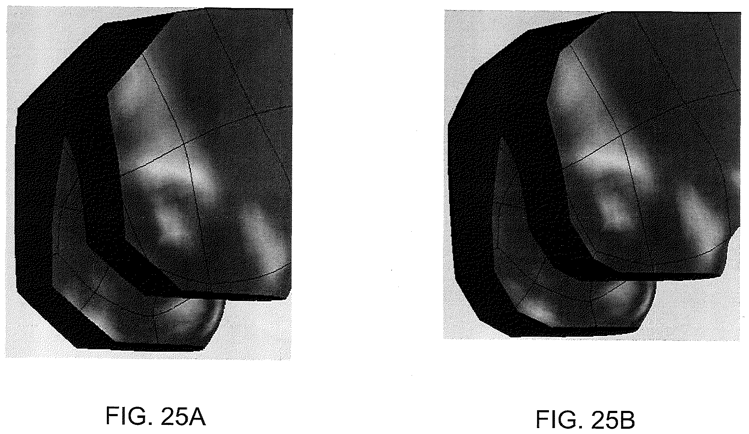

**FIG. 25A** shows a five-cut-plane femoral resection design for a femoral implant component having five bone cuts. **FIG. 25B** shows a seven-cut-plane femoral resection design for a femoral implant component having seven bone cuts. Each cut design was performed on the same patient femur model (i.e., having identical bone volumes). In addition, the corresponding five-bone-cut implant component and seven-bone-cut implant component were both designed meet or exceed the same minimum implant thickness. After performing the resection cuts, the model of the patient's femur having five resection cuts retained bone volume of 103,034 mm³, while the model of the patient's femur having seven bone cuts retained a bone volume of 104,220 mm³. As this analysis shows, the seven-bone-cut implant component saved substantially more of the patient's bone stock, in this case more than 1,000 mm³, as compared to the five-bone cut implant component.

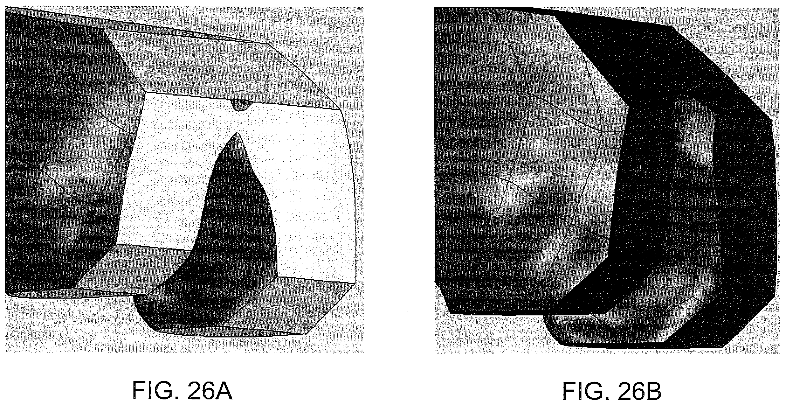

A similar analysis was performed to assess relative bone loss between a five-cut design and a five-flexed cut design. **FIG. 26A** shows a patient's femur having five, not flexed resection cuts and **FIG. 26B** shows the same femur but with five, flexed resection cuts. As shown, the model having five, not flexed resection cuts retains a bone volume of 109,472 mm³, while the model having five, flexed resection cuts retains a bone volume of 105,760 mm³. As this analysis shows, the not-flexed-five-bone-cut implant component saved substantially more of the patient's bone stock, in this case nearly 4,000 mm³, as compared to the flexed-five-bone-cut cut implant component. However, as noted in **Example 2,** the flexed cut design can offer other advantages, such as greater posterior coverage and enhanced deep-knee flexion, which can be weighed relative to all selected parameters and accordingly integrated in the selection and/or design of an implant component.

**FIGS. 27A** to **27D** show outlines of a traditional five-cut femoral component (in hatched lines) overlaid with, in **27A,** a femur having seven optimized resection cuts for matching an optimized seven-bone-cut implant component; in **FIG. 27B****,** a femur having five optimized resection cuts for matching to an optimized five-bone-cut implant component; in **FIG. 27C,** a femur having five, not flexed resection cuts for matching to an optimized five-bone-cut implant component; and in **FIG. 27D,** a femur having five, flexed resection cuts for matching to an optimized five-bone-cut, flexed implant component. As shown in each of these figures, the designed bone cuts save substantial bone as compared to those required by a traditional implant component.

In summary, the component designs described in this example can save patient bone stock as compared to a traditional implant component and thereby allow the implant to be pre-primary. Alternatively or in addition, the implant components may include cut planes (i.e., of resection cuts and bone cuts) that are optimized based on patient-specific data to meet one or more user-defined parameters, as described above. For example, cut planes can be symmetric or asymmetric, parallel or non-parallel, aligned perpendicular to the sagittal plane or not perpendicular, varied from medial to lateral condyle, and/or can include other orientations. The cut plane designs may include a "flexed" (i.e., rotated or offset relative to the biomechanical or anatomical axes) orientation. Moreover, the design of attachment pegs may also be flexed relative to the biomechanical or anatomical axes.

## Claims

1. A pre-primary articular implant component (500) for repairing a joint of a patient, said component comprising:
(a) an outer, joint-facing surface (504) comprising a bearing surface portion; and
(b) an inner, bone-facing surface (502) comprising one or more bone cut facets based on and/or designed from patient-specific data;
**characterized in that** the bearing surface portion has a sagittal profile derived from patient-specific data including image data of the joint of the patient.

2. The pre-primary articular implant component (500) of claim 1, wherein the bearing surface portion has a standard coronal profile.

3. The pre-primary articular implant component (500) of claim 2, wherein the standard coronal profile includes standard radii or curvature in one or more directions.

4. The pre-primary articular implant component (500) of claim 1, wherein the one or more patient-engineered bone cut facets are selected and/or designed from patient-specific data to minimize the amount of bone resected in one or more corresponding predetermined resection cuts, or
wherein the one or more patient-engineered bone cut facets substantially negatively-match one or more predetermined resection cuts,
wherein optionally the predetermined resection cuts are at a first depth that allows, in a subsequent procedure, removal of additional bone to a second depth required for a traditional primary implant component.

5. The pre-primary articular implant component (500) of claim 1, further comprising six or more patient-engineered bone cut facets, or
further comprising an implant component thickness in one or more regions that is selected and/or designed from patient-specific data to minimize the amount of bone resected,
wherein optionally the one or more regions comprises the implant component thickness perpendicular to a planar bone cut facet and between the planar bone cut facet and the joint-surface of the implant component.

6. A method for making an articular implant component (500) for a single patient in need of an articular implant replacement procedure, the method comprising:
(a) identifying unwanted tissue from one or more images of the patient's joint;
(b) identifying a combination of resection cuts and implant component features that remove the unwanted tissue and also provide maximum bone preservation; and
(c) selecting and/or designing a combination of resection cuts and/or implant component features that provide removal of the unwanted tissue and maximum bone preservation, comprising selecting and/or designing a patient-engineered or patient-specific sagittal profile of a bearing surface portion of an outer joint-facing surface of the articular implant component derived from patient specific data including image data of the joint of the patient,
optionally wherein step (c) comprises designing for the single patient a combination of resection cuts and implant component features that provide removal of the unwanted tissue and maximum bone preservation.

7. The method of claim 6, wherein step (c) further comprises selecting a standard coronal profile, or selecting and/or designing a patient-engineered or patient-specific coronal profile.

8. The method of claim 6, **characterized in that** the implant component features in step (c) include one or more of the features selected from the group consisting of implant thickness, bone cut number, bone cut angles, and/or bone cut orientations.

9. The method of claim 6, **characterized in that** a bone preservation measurement is selected from the group consisting of total volume of bone resected, volume of bone resected from one or more resection cuts, volume of bone resected to fit one or more implant component bone cuts, average thickness of bone resected, average thickness of bone resected from one or more resection cuts, average thickness of bone resected to fit one or more implant component bone cuts, maximum thickness of bone resected, maximum thickness of bone resected from one or more resection cuts, maximum thickness of bone resected to fit one or more implant component bone cuts.

10. The method of claim 6, **characterized in that** step (a) also includes identifying a minimum implant component thickness for the single patient, wherein optionally step (b) also includes identifying a combination of resection cuts and/or implant component features that provide a minimum implant thickness determined for the single patient, or wherein optionally step (c) includes selecting and/or designing the combination of resection cuts and/or implant component features that provides at least a minimum implant thickness for the single patient, or
wherein optionally the minimum implant component thickness is based on one or more of femur and/or condyle size or patient weight.

## Patentansprüche

1. Eine prä-primäre Gelenkimplantat-Komponente (500) zur Reparatur eines Gelenks eines Patienten, jene Komponente umfassend:
(a) eine äußere, gelenkzugewandte Oberfläche (504), umfassend einen tragenden Oberflächenanteil; und
(b) eine innere, knochenzugewandte Oberfläche (502), umfassend einen oder mehrere Knochenschnitt-Facetten, basierend auf patientenspezifischen Daten und/oder nach diesen gestaltet;
dadurch charakterisiert, dass der tragende Oberflächenanteil ein sagittales Profil hat, das aus patientenspezifischen Daten, umfassend Bilddaten des Gelenks des Patienten, abgeleitet ist.

2. Die prä-primäre Gelenkimplantat-Komponente (500) von Anspruch 1, wobei der tragende Oberflächenanteil ein standardmäßiges coronales Profil hat.

3. Die prä-primäre Gelenkimplantat-Komponente (500) von Anspruch 2, wobei das standardmäßige coronale Profil standardmäßige Radien oder Wölbung in einer oder mehreren Richtungen umfasst.

4. Die prä-primäre Gelenkimplantat-Komponente (500) von Anspruch 1, wobei die eine oder die mehreren für den Patienten konstruierten Knochenschnitt-Facetten nach patienten-spezifischen Daten ausgewählt und/oder gestaltet sind, um die Menge von Knochen, der in einem oder mehreren entsprechenden vorher festgelegten Resektionsschnitten reseziert wird, zu minimieren, oder wobei die eine oder die mehreren für den Patienten konstruierten Knochenschnitt-Facetten im Wesentlichen negativ mit einem oder mehreren vorherfestgelegten Resektionsschnitten übereinstimmen,
wobei die vorher festgelegten Resektionsschnitte optional bei einer ersten Tiefe sind, die, in einem folgenden Eingriff, die Entfernung von zusätzlichem Knochen bis zu einer zweiten Tiefe erlaubt, die für eine herkömmliche primäre Implantat-Komponente benötigt wird.

5. Die prä-primäre Gelenkimplantat-Komponente (500) von Anspruch 1, außerdem umfassend sechs oder mehr für den Patienten konstruierten Knochenschnitt-Facetten, oder
außerdem umfassend eine Implantat-Komponentendicke in einem oder mehreren Bereichen, die nach patientenspezifischen Daten ausgewählt oder gestaltet ist, um die Menge von reseziertem Knochen zu minimieren,
wobei der eine oder die mehreren Bereiche optional die Implantat-Komponentendicke senkrecht zu einer planraren Knochenschnitt-Facette und zwischen der planaren Knochenschnitt-Facette und der gelenkzugewandten Oberfläche der Implantat-Komponente umfasst.

6. Ein Verfahren zum Herstellung einer Gelenkimplantat-Komponente (500) für einen einzelnen Patienten, der einen Gelenkimplantat-Ersetzungseingriff benötigt, das Verfahren umfassend:
(a) Identifizieren von unerwünschtem Gewebe anhand eines oder mehrerer Bilder des Patientengelenks;
(b) Identifizieren einer Kombination von Resektionsschnitten und Implantat-Komponenten-Merkmalen, die das unerwünschte Gewebe entfernen und auch maximalen Knochenerhalt bieten; und
(c) Auswählen und/oder Gestalten einer Kombination von Resektionsschnitten und/oder Implantat-Komponenten-Merkmalen, die Entfernung des unerwünschten Gewebes und maximalen Knochenerhalt bieten, umfassend Auswählen und/oder Gestalten eines für den Patienten konstruierten oder patientenspezifischen sagittalen Profils eines tragenden Oberflächenanteils einer äußeren gelenkzugewandten Oberfläche der Gelenkimplantat-Komponente, das von patientenspezifischen Daten, umfassend Bilddaten des Gelenks des Patienten, abgeleitet ist,
wobei Schritt (c) optional das Gestalten einer Kombination von Resektionsschnitten und Implantat-Komponenten-Merkmalen umfasst, die Entfernung des unerwünschten Gewebes und maximalen Knochenerhalt bieten, für den einzelnen Patienten.

7. Das Verfahren von Anspruch 6, wobei Schritt (c) außerdem das Auswählen eines standardmäßigen coronalen Profils oder das Auswählen und/oder Gestalten eines für den Patienten konstruierten oder patientenspezifischen coronalen Profils umfasst.

8. Das Verfahren von Anspruch 6, dadurch charakterisiert, dass die Implantat-Komponenten-Merkmale in Schritt (c) eines oder mehrere der Merkmale, ausgewählt aus der Gruppe bestehend aus Implantat-Dicke, Knochenschnitt-Anzahl, Knochenschnitt-Winkeln und/oder Knochenschnitt-Orientierungen, umfassen.

9. Das Verfahren von Anspruch 6, dadurch charakterisiert, dass eine Knochenerhalt-Maßnahme aus der Gruppe bestehend aus totalem Volumen von reseziertem Knochen, Volumen von reseziertem Knochen von einem oder mehreren Resektionsschnitten, Volumen von reseziertem Knochen, um zu einem oder mehreren Implantat-Komponenten-Knochenschnitten zu passen, durchschnittlicher Dicke von reseziertem Knochen, durchschnittlicher Dicke von reseziertem Knochen von einem oder mehreren Resektionsschnitten, durchschnittlicher Dicke von reseziertem Knochen, um zu einem oder mehreren Implantat-Komponenten-Knochenschnitten zu passen, maximaler Dicke von reseziertem Knochen, maximaler Dicke von reseziertem Knochen von einem oder mehreren Resektionsschnitten, maximaler Dicke von reseziertem Knochen, um zu einer oder mehreren Implantat-Komponenten-Knochenschnitten zu passen, ausgewählt ist.

10. Das Verfahren von Anspruch 6, dadurch charakterisiert, dass Schritt (a) auch das Identifizieren einer minimalen Implantat-homponentendicke für den einzelnen Patienten umfasst, wobei Schritt (b) optional auch das Identifizieren einer Kombination von Resektionsschnitten und/oder Implantat-Komponenten-Merkmalen umfasst, die eine minimale Implantat-Dicke bieten, die für den einzelnen Patienten bestimmt wurde, oder wobei Schritt (c) optional das Auswählen und/oder Gestalten der Kombination von Resektionsschnitten und/oder Implantat-Komponenten-Merkmalen umfasst, die mindestens eine minimale Implantat-Dicke für den einzelnen Patienten bietet, oder wobei die minimale Implantat-Komponenten-Dicke auf einem oder mehreren von Femur- und/oder Kondylen-Größe oder Patientengewicht basiert.

## Revendications

1. Composant d'implant articulaire pré-primaire (500) pour la réparation d'une articulation d'un patient, ledit composant comprenant :
(a) une surface externe orientée vers l'articulation (504) comprenant une partie de surface de support ; et
(b) une surface interne orientée vers l'os (502) comprenant une ou plusieurs facettes de découpe d'os sur la base de et/ou conçues à partir de données spécifiques au patient ;
**caractérisé en ce que** la partie de surface de support présente un profil sagittal dérivé de données spécifiques au patient comportant des données image de l'articulation du patient.

2. Composant d'implant articulaire pré-primaire (500) selon la revendication 1, dans lequel la partie de surface de support présente un profil coronal normalisé.

3. Composant d'implant articulaire pré-primaire (500) selon la revendication 2, dans lequel le profil coronal normalisé comporte des rayons ou une courbure normalisés dans une ou plusieurs directions.

4. Composant d'implant articulaire pré-primaire (500) selon la revendication 1, dans lequel les unes ou plusieurs facettes de découpe d'os conçues en fonction du patient sont sélectionnées et/ou conçues à partir de données spécifiques au patient pour minimiser la quantité d'os réséquée dans une ou plusieurs coupes de résection prédéterminées correspondantes, ou dans lequel les unes ou plusieurs facettes de découpe d'os conçues en fonction du patient correspondent sensiblement de manière négative sensible à une ou plusieurs coupes de résection prédéterminées,
dans lequel optionnellement les coupes de résection prédéterminées sont à une première profondeur qui permet, lors d'une intervention subséquente, une extraction d'os supplémentaire à une seconde profondeur requise pour un composant d'implant primaire classique.

5. Composant d'implant articulaire pré-primaire (500) selon la revendication 1, comprenant en outre six, ou plus, facettes de découpe d'os conçues en fonction du patient, ou
comprenant en outre une épaisseur de composant d'implant dans une ou plusieurs régions qui est sélectionnée et/ou conçue à partir de données spécifiques au patient pour minimiser la quantité d'os réséquée,
dans lequel optionnellement les une ou plusieurs régions comprennent l'épaisseur de composant d'implant perpendiculaire à une facette plane de découpe d'os et entre la facette plane de découpe d'os et la surface d'articulation du composant d'implant.

6. Procédé de fabrication d'un composant d'implant articulaire (500) pour un patient unique nécessitant une intervention de remplacement d'implant articulaire, le procédé comprenant :
(a) l'identification de tissu indésirable à partir d'une ou plusieurs images de l'articulation du patient ;
(b) l'identification d'une combinaison de coupes de résection et de caractéristiques de composant d'implant qui extraient le tissu indésirable et fournissent également une préservation osseuse maximale ; et
(c) la sélection et/ou la conception d'une combinaison de coupes de résection et/ou de caractéristiques de composant d'implant qui fournissent l'extraction du tissu indésirable et une préservation osseuse maximale, comprenant la sélection et/ou la conception d'un profil sagittal conçu en fonction du patient ou spécifique au patient d'une partie de surface de support d'une surface externe orientée vers l'articulation du composant d'implant articulaire dérivé de données spécifiques au patient comportant des données image de l'articulation du patient,
optionnellement dans lequel l'étape (c) comprend la conception pour le patient unique d'une combinaison de coupes de résection et de caractéristiques de composant d'implant qui fournissent l'extraction du tissu indésirable et une préservation osseuse maximale.

7. Procédé selon la revendication 6, dans lequel l'étape (c) comprend en outre la sélection d'un profil coronal normalisé, ou la sélection et/ou la conception d'un profil coronal conçu en fonction du patient ou spécifique au patient.

8. Procédé selon la revendication 6, **caractérisé en ce que** les caractéristiques de composant d'implant à l'étape (c) comportent une ou plusieurs caractéristiques sélectionnées dans le groupe constitué d'une épaisseur d'implant, d'un nombre de découpes d'os, d'angles de découpe d'os, et/ou d'orientations de découpe d'os.

9. Procédé selon la revendication 6, **caractérisé en ce qu'**une mesure de préservation osseuse est sélectionnée dans le groupe constitué d'un volume total d'os réséqué, d'un volume d'os réséqué à partir d'une ou plusieurs coupes de résection, d'un volume d'os réséqué pour correspondre à une ou plusieurs découpes d'os de composant d'implant, d'une épaisseur moyenne d'os réséqué, d'une épaisseur moyenne d'os réséqué à partir d'une ou plusieurs coupes de résection, d'une épaisseur moyenne d'os réséqué pour correspondre à une ou plusieurs découpes d'os de composant d'implant, d'une épaisseur maximale d'os réséqué, d'une épaisseur maximale d'os réséqué à partir d'une ou plusieurs coupes de résection, d'une épaisseur maximale d'os réséqué pour correspondre à une ou plusieurs découpes d'os de composant d'implant.

10. Procédé selon la revendication 6, **caractérisé en ce que** l'étape (a) comporte également l'identification d'une épaisseur minimale de composant d'implant pour le patient unique, dans lequel optionnellement l'étape (b) comporte également l'identification d'une combinaison de coupes de résection et/ou de caractéristiques de composant d'implant qui fournissent une épaisseur minimale d'implant déterminée pour le patient unique, ou dans lequel optionnellement l'étape (c) comporte la sélection et/ou la conception de la combinaison de coupes de résection et/ou de caractéristiques de composant d'implant qui fournit au moins une épaisseur minimale d'implant pour le patient unique, ou dans lequel optionnellement l'épaisseur minimale de composant d'implant est basée sur un ou plusieurs parmi la taille du fémur et/ou du condyle ou le poids du patient.
